(19) Europäisches Patentamt
European Patent Office
Office européen des brevets

(11) **EP 2 006 687 A1**

(12) **EUROPEAN PATENT APPLICATION**

(43) Date of publication:
**24.12.2008 Bulletin 2008/52**

(51) Int Cl.:
***G01N 33/68*** *(2006.01)* *A61P 11/06* *(2006.01)*
*A61P 37/08* *(2006.01)*

(21) Application number: **07301135.5**

(22) Date of filing: **20.06.2007**

(84) Designated Contracting States:
**AT BE BG CH CY CZ DE DK EE ES FI FR GB GR
HU IE IS IT LI LT LU LV MC MT NL PL PT RO SE
SI SK TR**
Designated Extension States:
**AL BA HR MK RS**

(71) Applicant: **COMMISSARIAT A L'ENERGIE
ATOMIQUE
75015 Paris (FR)**

(72) Inventor: **The designation of the inventor has not
yet been filed**

(74) Representative: **Warcoin, Jacques et al
Cabinet Régimbeau
20, rue de Chazelles
75847 Paris cedex 17 (FR)**

(54) **Method of testing a subject thought to have or be predisposed to having asthma, allergie,
atopic disease or atopic sensitization**

(57) The present invention concerns a method of testing a subject thought to have or be predisposed to having asthma, allergic, atopic disease or atopic sensitization, which comprises the step of analyzing a biological sample from said subject for (i) detecting the presence of a mutation associated with the over-expression of a gene of the ORM1-like gene (ORMDL) family, and/or (ii) analyzing the expression of a gene of the ORMDL family; a use, for treating and/or preventing asthma, allergic, atopic disease or atopic sensitization in a subject, of a compound which specifically inhibits the expression of a gene of the ORMDL family; and an *in vitro* method of selecting a compound, which can be useful for treating asthma, allergie, atopic disease or atopic sensitization, characterized in that said method comprises the steps of: (a) obtaining a cell expressing a gene of the ORMDL family, (b) contacting said cell with at least one compound, (c) comparing the expression of the gene of the ORMDL family in the cell between the steps a) and b), and (d) selecting the compound, which induces a lower level of expression of the gene of the ORMDL family in the cell contacted to that compound.

**Description**

<u>Field of the invention</u>

The present invention relates to asthma, allergies, atopic diseases or atopic sensitization, and more precisely to a method of testing a subject thought to have or to be predisposed to asthma, allergies, atopic diseases or atopic sensitization.

<u>Background of the invention</u>

Asthma is a chronic inflammatory disorder and, in genetically-susceptible individuals, this inflammation leads to increased airway responsiveness to a variety of stimuli, and recurrent airway obstruction. It is the most common chronic disease of childhood and the most common reason for pediatric hospital admission. Although it is clear that both environmental and genetic influences are important in the development of asthma, the pathogenesis of this disease remains unclear.

Although genes associated with asthma or atopy have been found (OBER & HOFFJAN, Genes Immun., vol. 7, p:95-100, 2006), including IL-4, HLA complex, FcgR1ss, ss2 adrenergic receptor, only a small proportion of the susceptibility factors have so far been identified from regions of linkage (COOKSON & MOFFATT, N. Engl. J. Med., vol. 351, p:1794-6, 2004). Delineating the genes which contribute to the development of asthma, and dissecting the mechanisms by which these genes alter the host response to environmental challenge (antigen, viral, etc.) are key steps to furthering our knowledge of the pathogenesis of asthma.

Despite advances in genotyping, the statistical power to identify genetic effects is constrained by sample size. Power may be enhanced by the study of intermediate phenotypes, in particular transcript abundances of genes (expression QTLs) that may be directly modified by polymorphism in regulatory elements (SCHADT et al., Nature, vol.422, p: 297-302, 2003; MORLEY et al., Nature, vol.430, p:743-7, 2004).

Previous studies have shown the potential for eQTL mapping (SCHADT *et al.,* 2003, abovementioned ; MORLEY *et al.,* 2004, abovementioned ; CHEUNG et al., Nat. Genet., vol.33, p:422-5, 2003), but have examined limited numbers of transcripts or markers in a small number of CEPH (Centre d'Etude du Polymorphisme Humain) pedigrees. The EBV-transformed lymphoblastoid cell lines (EBVL) in these experiments had undergone multiple cell culture passages, which increases the risk of covert chromosomal rearrangements.

Because asthma can become progressively more severe over time, it is important to determine individuals that are susceptible to the disease at a young age. Thus, what is needed in the art is a mechanism for determining whether an individual is at risk for asthma. The present invention fulfils such a need.

<u>Summary of the invention</u>

The present invention provides a method of testing a subject thought to have or be predisposed to having asthma, allergie, atopic disease or atopic sensitization which comprises the step of analyzing a biological sample from said subject for:

(i) detecting the presence of a mutation associated with the over-expression of a gene of the ORM1-like gene (ORMDL) family, and/or

(ii) analyzing the expression of a gene of the ORMDL family.

In another embodiment, the present invention provides also a use, for treating and/or preventing asthma, allergie, atopic disease or atopic sensitization in a subject, of a compound which specifically inhibits the expression of a gene of the ORMDL family.

In still another embodiment, the present invention provides an *in vitro* method of selecting a compound, which can be useful for treating asthma, allergie, atopic disease or atopic sensitization, characterized in that said method comprises the steps of:

a) obtaining a cell expressing a gene of the ORMDL family,

b) contacting said cell with at least one compound,

c) comparing the expression of the gene of the ORMDL family in the cell between the steps a) and b), and

d) selecting the compound, which induces a lower level of expression of the gene of the ORMDL family in the cell contacted to that compound.

Brief description of the drawings

Figurela shows the distribution of total heritability for expression quantitative traits.
The figure 1b shows the distribution of lod scores for association between 14819 traits and 408,273 SNP markers.
The figure 1c shows the proportion of traits with peak LOD > 6 according to total trait heritability.
The figure 1d shows the total heritability accounted for by the SNP showing strongest association with gene expression levels.
The figure 2a shows the mapping of genes with GO-BP descriptors for cell cycle, DNA repair and RNA processing.
The Figure 2b shows the mapping of 21 genes with GO-DP descriptors for immune responses.
The figure 3 shows the genome-wide association of 317,447 SNPs and asthma in 994 asthmatic children and 1,244 non-asthmatic children.
The figure 4 shows the aassociation to asthma and transcript abundances of *ORMDL3* on chromosome 17q21.

Detailed description of the invention

The present invention is based on the discovery by the present inventors that an over-expression of the ORMDL3 transcripts and particular single nucleotide polymorphisms (SNP), which are associated with the over-expression of the ORMDL3 gene, are both correlated with asthma.
Thus, in a first aspect, the present invention provides a method of testing a subject thought to have or be predisposed to having asthma, allergie, atopic disease or atopic sensitization which comprises the step of analyzing a biological sample from said subject for :

(i) detecting the presence of a mutation associated with the over-expression of a gene of the ORM1-like gene (ORMDL) family, and/or

(ii) analyzing the expression of a gene of the ORMDL family.

Preferably, said method is useful for of testing a subject thought to have or be predisposed to having asthma.
Said method, by identifying an over-expression of a gene of the ORMDL family and/or by detecting the presence of a mutation associated with the over-expression of a gene of the ORM1-like gene (ORMDL) family in a subject enables to confirm that said subject has or is predisposed for having asthma.
As used herein, the term "subject" refers to a mammal, preferably a human.
As used herein, the expression "biological sample" refers to solid tissues such as, for example, a lung biopsy; buccal swab, fluids and excretions such as for example, sputum, induced sputum, blood, serum, plasma, urine. Preferably, said biological sample is a fluid sample and most preferably a blood sample.
As used herein, the expression "gene of the ORM1-like gene (ORMDL) family" refers to a conserved new family of endoplasmic reticulum membrane proteins, which has been described in HJELMQVIST et al. (Genome biology, vol. 3(6), RESEARCH0027, 2002). The ORMDL family includes the three ORMDL1, ORMDL2 and ORMDL3 genes in mammals. The sequences of the genes of the ORMDL family are well known by one of skill in the art and includes the gene ORMDL1 for *Homo sapiens* (mRNA: AF395704, protein: AAM43503), *Mus musculus* (mRNA: BCO23695, protein: AAH23695), *Bos Taurus* (mRNA: NM_001014931, protein: NP_001014931), *Pongo pygmaeus* (mRNA: CR859623, protein: Q5R8X5), and *Macaca mulatta* (mRNA: XM_001107686, protein: XP_001107686); the gene ORMDL2 for *Homo sapiens* (mRNA: NM_014182, protein: NP_054901), *Mus musculus* (mRNA: BC002146, protein: AAH02146), *Bos Taurus* (mRNA: BT021048, protein: AAX09065), and *Pan troglodytes* (mRNA: XM_509124, protein: XP_ 509124); and the gene ORMDL3 for *Homo sapiens* (mRNA: NM_139280, SEQ ID NO: 1, protein: NP_644809, SEQ ID NO:2, gene: corresponds to position 35330671 to 35331488 from NC_000017.9), *Mus musculus* (mRNA: BC026412, protein: AAH26412), *Pan troglodytes* (mRNA: XM_001171472, protein: XP_001171472), *Pongo pygmaeus* (mRNA: CR860995, protein: Q5R570), and *Rattus norvegicus* (mRNA: AY539934, protein: Q6QI25).
Preferably, said gene of the ORM1-like gene (ORMDL) family is the ORMDL3 gene.
Typically, the mutation (s) associated with the over-expression of a gene of the ORM1-like gene (ORMDL) family corresponds to a single nucleotide polymorphism (SNP).
Preferably, said mutation(s) associated with the over-expression of a gene of the ORM1-like gene (ORMDL) family has the same chromosomal location as said gene.

As an example and for a human subject, said mutation can be located on chromosome 2q32, on chromosome 12q13, or on chromosome 17q21, when said mutation is associated with an over-expression of the ORMDL1, ORMDL2 or ORMDL3 gene respectively.

Preferably, said mutation is a single nucleotide polymorphism associated with the over-expression of the ORMDL3 gene in a human, wherein said mutation is located on chromosome 17q21 and is selected from the group comprising *rs9303277* (nucleotide N at position 128 of SEQ ID NO:3, wherein allele C is associated to asthma), *rs11557467* (nucleotide N at position 128 of SEQ ID NO:4, wherein allele G is associated to asthma), *rs8067378* (nucleotide N at position 128 of SEQ ID NO:5, wherein allele A is associated to asthma), *rs2290400* (nucleotide N at position 128 of SEQ ID NO:6, wherein allele A is associated to asthma), *rs7216389* (nucleotide N at position 128 of SEQ ID NO:7, wherein allele T is associated to asthma), *rs4795405* (nucleotide N at position 128 of SEQ ID NO:8, wherein allele C is associated to asthma), *rs8079416* (nucleotide N at position 128 of SEQ ID NO:9, wherein allele C is associated to asthma), *rs4795408* (nucleotide N at position 128 of SEQ ID NO:10, wherein allele A is associated to asthma), *rs3894194* (nucleotide N at position 128 of SEQ ID NO:11, wherein allele A is associated to asthma), *rs3859192* (nucleotide N at position 128 of SEQ ID NO: 12, wherein allele T is associated to asthma), rs9646419 (nucleotide N at position 128 of SEQ ID NO:13, wherein allele A is associated to asthma), rs14050 (nucleotide N at position 128 of SEQ ID NO:14, wherein allele C is associated to asthma), rs2941503 (nucleotide N at position 128 of SEQ ID NO:15, wherein allele A is associated to asthma), rs907087 (nucleotide N at position 128 of SEQ ID NO:16, wherein allele G is associated to asthma), rs2517954 (nucleotide N at position 128 of SEQ ID NO:17, wherein allele T is associated to asthma), rs1810132 (nucleotide N at position 128 of SEQ ID NO:18, wherein allele C is associated to asthma), rs907091 (nucleotide N at position 128 of SEQ ID NO:19, wherein allele T is associated to asthma), rs907092 (nucleotide N at position 128 of SEQ ID NO:20, wherein allele G is associated to asthma), rs10445308 (nucleotide N at position 128 of SEQ ID NO: 21, wherein allele C is associated to asthma), rs10852936 (nucleotide N at position 128 of SEQ ID NO:22, wherein allele C is associated to asthma), rs1054609 (nucleotide N at position 128 of SEQ ID NO:23, wherein allele A is associated to asthma), rs8067378 (nucleotide N at position 128 of SEQ ID NO:24, wherein allele A is associated to asthma), rs2123685 (nucleotide N at position 128 of SEQ ID NO:25, wherein allele C is associated to asthma), rs8069176 (nucleotide N at position 128 of SEQ ID NO:26, wherein allele G is associated to asthma), rs2305480 (nucleotide N at position 128 of SEQ ID NO:27, wherein allele G is associated to asthma), rs2305479 (nucleotide N at position 128 of SEQ ID NO:14, wherein allele C is associated to asthma), rs11078926 (nucleotide N at position 128 of SEQ ID NO:29, wherein allele G is associated to asthma), rs1008723 (nucleotide N at position 128 of SEQ ID NO:30, wherein allele G is associated to asthma), rs4795400 (nucleotide N at position 128 of SEQ ID NO:31, wherein allele C is associated to asthma), rs7216389 (nucleotide N at position 128 of SEQ ID NO:32, wherein allele T is associated to asthma), rs9303281 (nucleotide N at position 128 of SEQ ID NO:33, wherein allele A is associated to asthma), rs7219923 (nucleotide N at position 128 of SEQ ID NO:34, wherein allele T is associated to asthma), rs3169572 (nucleotide N at position 128 of SEQ ID NO:35, wherein allele A is associated to asthma), rs4378650 (nucleotide N at position 128 of SEQ ID NO:36, wherein allele G is associated to asthma), rs8076131 (nucleotide N at position 128 of SEQ ID NO:37, wherein allele A is associated to asthma), rs3744246 (nucleotide N at position 128 of SEQ ID NO:38, wherein allele C is associated to asthma), rs4795402 (nucleotide N at position 128 of SEQ ID NO:39, wherein allele C is associated to asthma), rs4795403 (nucleotide N at position 128 of SEQ ID NO:40, wherein allele C is associated to asthma), rs4795404 (nucleotide N at position 128 of SEQ ID NO:41, wherein allele C is associated to asthma), rs4795405 (nucleotide N at position 128 of SEQ ID NO:42, wherein allele C is associated to asthma), rs4794820 (nucleotide N at position 128 of SEQ ID NO:43, wherein allele G is associated to asthma), rs7207600 (nucleotide N at position 128 of SEQ ID NO:44, wherein allele A is associated to asthma), rs6503525 (nucleotide N at position 128 of SEQ ID NO:45, wherein allele C is associated to asthma), rs8065126 (nucleotide N at position 128 of SEQ ID NO:46, wherein allele C is associated to asthma), rs3893044 (nucleotide N at position 128 of SEQ ID NO:47, wherein allele C is associated to asthma), rs4795408 (nucleotide N at position 128 of SEQ ID NO:48, wherein allele A is associated to asthma), rs7209742 (nucleotide N at position 128 of SEQ ID NO:49, wherein allele G is associated to asthma), rs8076474 (nucleotide N at position 128 of SEQ ID NO:50, wherein allele C is associated to asthma), rs1007654 (nucleotide N at position 128 of SEQ ID NO:51, wherein allele G is associated to asthma), rs1007655 (nucleotide N at position 128 of SEQ ID NO:52, wherein allele A is associated to asthma), rs2313640 (nucleotide N at position 128 of SEQ ID NO:53, wherein allele T is associated to asthma), rs7218742 (nucleotide N at position 128 of SEQ ID NO:54, wherein allele G is associated to asthma), rs7218321 (nucleotide N at position 128 of SEQ ID NO:55, wherein allele T is associated to asthma), rs7219080 (nucleotide N at position 128 of SEQ ID NO:56, wherein allele C is associated to asthma), rs6503526 (nucleotide N at position 128 of SEQ ID NO:57, wherein allele T is associated to asthma), rs6503527 (nucleotide N at position 128 of SEQ ID NO:58, wherein allele A is associated to asthma), rs3894194 (nucleotide N at position 128 of SEQ ID NO:59, wherein allele A is associated to asthma), rs7212938 (nucleotide N at position 128 of SEQ ID NO:60, wherein allele T is associated to asthma), rs2305479 (nucleotide N at position 128 of SEQ ID NO:61, wherein allele C is associated to asthma), rs2305480 (nucleotide N at position 128 of SEQ ID NO:62, wherein allele G is associated to asthma), and rs2941503 (nucleotide N at position 128 of SEQ ID NO:63, wherein allele A is associated to asthma).

Most preferably, said mutation is a single nucleotide polymorphism associated with the over-expression of the ORMDL3 gene in a human, wherein said mutation is located on chromosome 17q21 and is selected from the group comprising *rs9303277* (nucleotide N at position 128 of SEQ ID NO:3, wherein allele C is associated to asthma), *rs11557467* (nucleotide N at position 128 of SEQ ID NO:4, wherein allele G is associated to asthma), *rs8067378* (nucleotide N at position 128 of SEQ ID NO:5, wherein allele A is associated to asthma), *rs2290400* (nucleotide N at position 128 of SEQ ID NO:6, wherein allele A is associated to asthma), *rs7216389* (nucleotide N at position 128 of SEQ ID NO:7, wherein allele T is associated to asthma), *rs4795405* (nucleotide N at position 128 of SEQ ID NO:8, wherein allele C is associated to asthma), *rs8079416* (nucleotide N at position 128 of SEQ ID NO:9, wherein allele C is associated to asthma), *rs4795408* (nucleotide N at position 128 of SEQ ID NO:10, wherein allele A is associated to asthma), *rs3894194* (nucleotide N at position 128 of SEQ ID NO:11, wherein allele A is associated to asthma), and *rs3859192* (nucleotide N at position 128 of SEQ ID NO: 12, wherein allele T is associated to asthma).

The skilled man will immediately appreciate that the information presented herein relating to the human ORMDL3 may easily be equated or correlated with a similar mutation at a corresponding location for the ORMDL3 gene from another species.

Typical techniques for detecting the mutation may include restriction fragment length polymorphism, hybridisation techniques, DNA sequencing, exonuclease resistance, microsequencing, solid phase extension using ddNTPs, extension in solution using ddNTPs, oligonucleotide ligation assays, methods for detecting single nucleotide polymorphisms such as dynamic allele-specific hybridisation, ligation chain reaction, mini-sequencing, DNA"chips", allele-specific oligonucleotide hybridisation with single or dual-labelled probes merged with PCR or with molecular beacons, and others.

Preferably, said technique for detecting a mutation is selected in the group comprising methods for detecting single nucleotide polymorphisms.

Analyzing the expression of a gene of the ORMDL family may be assessed by any of a wide variety of well-known methods for detecting expression of a transcribed nucleic acid or translated protein.

In a preferred embodiment, the expression of a gene of the ORMDL family is assessed by analyzing the expression of mRNA transcript or mRNA precursors, such as nascent RNA, of said gene. Said analysis can be assessed by preparing mRNA/cDNA from cells in a biological sample from a subject, and hybridizing the mRNA/cDNA with a reference polynucleotide. The prepared mRNA/cDNA can be used in hybridization or amplification assays that include, but are not limited to, Southern or Northern analyses, polymerase chain reaction analyses, such as quantitative PCR (TaqMan), and probes arrays such as GeneChip™ DNA Arrays (AFFYMETRIX).

Advantageously, the analysis of the expression level of mRNA transcribed from a gene of the ORMDL family involves the process of nucleic acid amplification, e. g., by RT-PCR (the experimental embodiment set forth in U. S. Patent No. 4,683, 202), ligase chain reaction (BARANY, Proc. Natl. Acad. Sci. USA, vol.88, p: 189-193, 1991), self sustained sequence replication (GUATELLI et al., Proc. Natl. Acad. Sci. USA, vol.87, p: 1874-1878, 1990), transcriptional amplification system (KWOH et al., 1989, Proc. Natl. Acad. Sci. USA, vol.86, p: 1173-1177, 1989), Q-Beta Replicase (LIZARDI et al., Biol. Technology, vol.6, p: 1197, 1988), rolling circle replication (U. S. Patent No. 5,854, 033) or any other nucleic acid amplification method, followed by the detection of the amplified molecules using techniques well known to those of skill in the art. These detection schemes are especially useful for the detection of nucleic acid molecules if such molecules are present in very low numbers. As used herein, amplification primers are defined as being a pair of nucleic acid molecules that can anneal to 5' or 3'regions of a gene (plus and minus strands, respectively, or vice-versa) and contain a short region in between. In general, amplification primers are from about 10 to 30 nucleotides in length and flank a region from about 50 to 200 nucleotides in length. Under appropriate conditions and with appropriate reagents, such primers permit the amplification of a nucleic acid molecule comprising the nucleotide sequence flanked by the primers.

In another preferred embodiment, the expression of a gene of the ORMDL family is assessed by analyzing the expression of the protein translated from said gene. Said analysis can be assessed using an antibody (e.g., a radio-labeled, chromophore-labeled, fluorophore-labeled, or enzyme-labeled antibody), an antibody derivative (e.g., an antibody conjugate with a substrate or with the protein or ligand of a protein of a protein/ligand pair (e.g., biotin-streptavidin)), or an antibody fragment (*e.g.*, a single-chain antibody, an isolated antibody hypervariable domain, *etc.)* which binds specifically to the protein translated from a gene of the ORMDL family.

Said analysis can be assessed by a variety of techniques well known by one of skill in the art including, but not limited to, enzyme immunoassay (EIA), radioimmunoassay (RIA), Western blot analysis and enzyme linked immunoabsorbant assay (ELISA).

Polyclonal antibodies can be prepared by immunizing a suitable animal, such as mouse, rabbit or goat, with a protein encoded by a gene of the ORMDL family or a fragment thereof. The antibody titer in the immunized animal can be monitored over time by standard techniques, such as with an ELISA using immobilized polypeptide. At an appropriate time after immunization, e.g., when the specific antibody titers are highest, antibody producing cells can be obtained from the animal and used to prepare monoclonal antibodies (mAb) by standard techniques, such as the hybridoma technique originally described by KOHLER and MILSTEIN (Nature, vol.256, p:495-497, 1975), the human B cell hybridoma technique (KOZBOR et al., Immunol., vol.4, p: 72, 1983), the EBV- hybridoma technique (COLE et al., In Monoclonal Antibodies and Cancer Therapy, Alan R. Liss,Inc., p: 77-96, 1985) or trioma techniques. The technology for producing hybridomas is well known (see generally Current Protocols in Immunology, COLIGAN et al. ed. , John Wiley & Sons, New York, 1994). Hybridoma cells producing the desired monoclonal antibody are detected by screening the hybridoma culture supernatants for antibodies that bind the polypeptide of interest, e.g., using a standard ELISA.

The method of the invention may comprise comparing the level of expression of a gene of the ORMDL family in a biological sample from a subject with the normal expression level of said gene in a control. A significantly higher level of expression of said gene in the biological sample of a subject as compared to the normal expression level is an indication that the patient has or is predisposed to asthma.

An "over-expression" of a gene of the ORMDL family refers to an expression level in a biological sample that is greater than the standard error of the assay employed to assess expression, and is preferably at least 20% superior to the normal level of expression of said gene, preferably at least 50% superior to the normal level of expression of said gene, and most preferably at least 100% superior to the normal level of expression of said gene.

The "normal" level of expression of a gene of the ORMDL family is the level of expression of said gene in a biological sample of a subject not afflicted with asthma. Preferably, said normal level of expression is assessed in a control sample (e.g., sample from a healthy subject, which is not afflicted by asthma) and preferably, the average expression level of said gene in several control samples.

In another aspect, the present invention provides a use, for treating and/or preventing asthma, allergie, atopic disease or atopic sensitization in a subject, of a compound which specifically inhibits the expression of a gene of the ORMDL family.

Preferably, the present invention provides a use, for treating and/or preventing asthma in a subject, of a compound which specifically inhibits the expression of a gene of the ORMDL family.

Preferably, said compound specifically inhibiting the expression a gene of the ORMDL family is an oligonucleotide, which is selected from the group comprising anti-sense RNA and DNA molecules, ribozymes, siRNAs and aptamers.

More specifically, anti-sense RNA and DNA molecules and ribozymes function to inhibit the translation of ORMDL mRNA. Anti-sense RNA and DNA molecules act to directly block the translation of mRNA by binding to targeted mRNA and preventing protein translation. With regard to anti-sense DNA, oligodeoxyribonucleotides derived from the translation initiation site, e. g. , between - 10 and +10 regions of the ORMDL3 nucleotide sequence, are preferred. Ribozymes are enzymatic RNA molecules capable of catalyzing the specific cleavage of RNA. The mechanism of ribozyme action involves sequence specific hybridization of the ribozyme molecule to complementary target RNA, followed by a endonucleolytic cleavage. Within the scope of the invention are engineered hammerhead motif ribozyme molecules that specifically and efficiently catalyze endonucleolytic cleavage of ORMDL RNA sequences, preferably ORMDL3 RNA sequences. Specific ribozyme cleavage sites within any potential RNA target are initially identified by scanning the target molecule for ribozyme cleavage sites which include the following sequences, GUA, GUU and GUC. Once identified, short RNA sequences of between 15 and 20 ribonucleotides corresponding to the region of the target gene containing the cleavage site may be evaluated for predicted structural features such as secondary structure that may render the oligonucleotide sequence unsuitable. Both anti-sense RNA and DNA molecules and ribozymes of the invention may be prepared by any method known in the art for the synthesis of RNA molecules. These include techniques for chemically synthesizing oligodeoxyribonucleotides well known in the art such as for example solid phase phosphoramidite chemical synthesis. Alternatively, RNA molecules may be generated by in vitro and in vivo transcription of DNA sequences encoding the anti-sense RNA molecule.

Such DNA sequences may be incorporated into a wide variety of vectors which incorporate suitable RNA polymerase promoters such as the T7 or SP6 polymerase promoters. Alternatively, anti-sense cDNA constructs that synthesize anti-sense RNA constitutively or inducibly, depending on the promoter used, can be introduced stably into cell lines.

Short interference RNA molecules (siRNA) can also be used for inhibiting the expression of a gene of the ORMDL family. Said interference RNA molecules can be generated based on the genetic sequences of ORMDL genes, preferably ORMDL3. RNA interference (RNAi) is based on the degradation of particular target sequences by the design of short interference RNA oligo's (siRNA) which recognize the target sequence (here ORMDL) and subsequently trigger their degradation by a poorly understood pathway. In general siRNA duplexes are shorter than 30 nucleotides, because longer stretches of dsRNA activate the PKR pathway in mammalian cells which results in a global a-specific shut-down of protein synthesis. Preferably, the length of said siRNA is comprised between 15 and 25 bp (bases pair), and most preferably between 19 and 24 bp. The preparation and gene therapy vectors for the intracellular expression of siRNAs duplexes is disclosed in W00244321. As an example of siRNA, one can cite SEQ ID NO:64 (CUAAGUACGACCAGAUCCA), SEQ ID NO: 65 (AAGGCAUGUGCUGCAACAC), SEQ ID NO: 66 (AGAAGAAGCCUCUGGACAC), SEQ ID NO: 67 (GUAGCCAACUUGGAGUAGC), SEQ ID NO: 68 (UCAAUAAGUACUGAGAGUG), SEQ ID NO: 69 (UAAGUACUGAGAGUGCAGC), and SEQ ID NO: 70 (AGUUCUUGACCAUCACACC).

RNA aptamers can also be used for inhibiting the expression of a gene of the ORMDL family. Said RNA aptamers can be generated against ORMDL genes, preferably ORMDL3. Recently, RNA aptamers have been used as therapeutic reagents for their ability to disrupt protein function. Selection of aptamers *in vitro* allows rapid isolation of extremely rare RNAs that have high specificity and affinity for specific proteins. Exemplary RNA aptamers are described in U. S. Pat. No. 5,270,163, in GOLD et al. (Nature, vol.346, p:818-822, 1990), and TUERK & GOLD (Science, vol.249, p:505-510, 1990). Unlike anti-sense compounds, whose targets are one dimensional lattices, RNA aptamers can bind to the three dimensional surfaces of a protein. Moreover, RNA aptamers can frequently discriminate finely among discrete functional sites of a protein (GOLD et al., Annu. Rev. Biochem., vol.64, p:763-797, 1995). As research and therapeutic reagents, aptamers not only have the combined advantages of antibodies and small molecular mass drugs, but in vivo production of RNA aptamers also can be controlled genetically. Such RNA expressing genes are usually smaller than protein-coding genes and can be inserted easily into gene therapy vectors.

Preferably, said oligonucleotide is a siRNA.

The oligonucleotide may be delivered *in vivo* alone or in association with a vector. In its broadest sense, a "vector" is any vehicle capable of facilitating the transfer of the siRNA to the cells and preferably cells over-expressing a gene of the ORMDL family, such as PBMC.

As an example, such oligonucleotide can be associated with non-lipid cationic polymers (WU and WU, J. Biol. Chem., vol.263, p: 14621-4, 1988) or liposomes (BRIGHMAN et al., Am. J. Med. Sci., vol.298, p: 278-81, 1989) to form complexes enhancing cellular uptake.

Advantageously, said compound specifically inhibiting the expression a gene of the ORMDL family may be associated with a pharmaceutically acceptable vehicle.

As an example of pharmaceutically acceptable vehicle, the composition may comprise emulsions, microemulsions, oil-in-water emulsions, anhydrous lipids and oil-in-water emulsions, other types of emulsions. The composition may also comprise one or more additives (e.g., diluents, excipients, stabilizers, preservatives). See, generally, Ullmann's Encyclopedia of Industrial Chemistry, 6th Ed. (various editors, 1989-1998, Marcel Dekker); and Pharmaceutical Dosage Forms and Drug Delivery Systems (ANSEL et al., 1994, WILLIAMS & WILKINS).

In still another aspect, the present invention provides an *in vitro* method of selecting a compound, which can be useful for treating asthma, allergie, atopic disease or atopic sensitization, characterized in that said method comprises the steps of:

a) obtaining a cell expressing a gene of the ORMDL family;

b) contacting said cell with at least one compound,

c) comparing the expression of the gene of the ORMDL family in the cell between the steps a) and b),

d) selecting the compound, which induces a lower level of expression of the gene of the ORMDL family in the cell contacted to that compound.

Preferably, said method is useful for selecting a compound, which can be useful for treating asthma.

Preferably, said gene of the ORMDL family corresponds to ORMDL3.

Advantageously, said cell expressing a gene of the ORMDL family has been obtained from a subject suffering from asthma or has been derivated from a cell obtained from a subject suffering from asthma.

Preferably, said cell over-expressed the ORMDL3 gene, as compared to the corresponding cells from a subject which is not suffering from asthma.

Preferably, said cell corresponds to a peripheral blood leukocyte (PBL), and most preferably to a peripheral blood leukocyte immortalized with Epstein Barr Virus.

As used herein, the term "compound" refers to any type of molecules such as polypeptides, polynucleotides, sugars, lipids, or any other chemical compounds.

Methods for determining the expression of a gene of the ORMDL family are well known from one of skill in the art. As an example, one can cite the methods which have been described previously.

In the following, the invention is described in more detail with reference to amino acid sequences, nucleic acid sequences and the examples. Yet, no limitation of the invention is intended by the details of the examples. Rather, the invention pertains to any embodiment which comprises details which are not explicitly mentioned in the examples herein, but which the skilled person finds without undue effort.

Examples

1) Subjects:

We recruited a panel of 206 nuclear families (MRC-A) through a proband with severe (Step III) childhood asthma as part of the MRC UK National family collection. We included siblings regardless of asthma status [6].

Children and their parents from the UK panels were administered a standard questionnaire (based on the American Thoracic Society and ISAAC questionnaires; Standards for the diagnosis and care of patients with chronic obstructive pulmonary disease (COPD) and asthma. This official statement of the American Thoracic Society was adopted by the ATS Board of Directors, November 1986. Am. Rev. Respir. Dis., vol.136, p: 225-44, 1987) by a nurse practitioner or a doctor. Asthma was defined as a positive response to the question "Has your doctor ever told you that you have asthma". Probands had Step 3 asthma or worse according to the BTS guidelines (high dose inhaled steroids, or low dose inhaled steroids and a long-acting β-agonist; British guideline on the management of asthma, Thorax, vol.58 (Suppl 1), p:il-94, 2003).

All study methods were approved by the appropriate ethics committees.

2) EBVL establishing:

EBVL were established on probands and siblings. The transformation of the PBLs (Peripheral Blood Leukocytes) in all children in the panel was carried out by the ECACC (European Collection of Cell cultures). Previously transformed cryo-preserved EBV cell lines were grown as 500 ml roller cultures. Once log phase had been obtained, cells were pelleted, media discarded and a mixture of RLT buffer and β-mercaptoethanol added. Pellets were vortexed to ensure thorough re-suspension after which they were frozen at -70°C and kept at -80°C. RNA was extracted in batches after cell homogenization using RNeasy® Maxi Kits (QIAGEN) and quality and quantity assessed.

3) Gene expression:

Global gene expression was measured.

10 µg of previously purified RNA was used to synthesize double-stranded cDNA using the One-cycle cDNA synthesis kit (AFFYMETRIX). Using the cDNA as a template, *in vitro* transcription of cRNA was carried out using the IVT kit (AFFYMETRIX) following the manufacturer's protocol. A hybridization cocktail was made according to protocol, using 15µg of labelled, fragmented cRNA and hybridized to U133 Plus 2.0 GeneChips (AFFYMETRIX) for 16 hours at 45°C in a rotating oven. GeneChips were washed and stained following the protocol and scanned on a high-resolution scanner (AGILENT TECHNOLOGIES).

All data from the gene expression experiment was normalized together using the RMA (Robust Multi-Array Average) package (IRIZARRY et al., Biostatistics, vol.4, p:249-64, 2003; BOLSTAD et al., Bioinformatics, vol.19, p: 185-93, 2003) to remove any technical or spurious background variation. An inverse normalization transformation step was also applied to each trait to avoid any outliers. A variance components method was used to estimate heritability of each trait using the Merlin-regress (RandomSample option; ABECASIS et al., Nat. Genet., vol.30, p:97-101, 2002; SHAM et al., Am. J. Hum. Genet., vol.71, p:238-53, 2002).

Gene locations (to separate cis and trans-associations) were extracted from the UCSC table browser available at www.ncbi.nlm.nih.gov/RefSeq/. Annotations for individual transcripts on the Affymetrix arrays were extracted from the Affymetrix NetAffx database available at www.affymetrix.com.

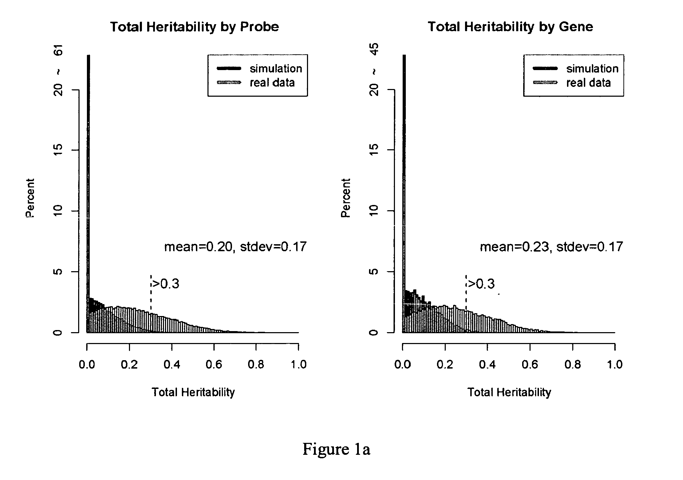

Figure 1a shows the distribution of total heritability for expression quantitative traits, wherein a reference null distribution was generated by permuting phenotypes across families. The left panel shows heritability estimates for each probe. In the right panel, expression measurements were averaged for all probes in the same gene before calculating heritability.

The results show that the $H^2$ for all the traits ranged between 0.0 - 1.0, with a mean of 0.203 and a 3rd quartile (Q3) of 0.317 (Figure 1a).

We focused subsequent analyses on 15,084 traits with $H^2 > 0.3$.

4) Genotyping:

Whole genome genotyping (WGGT) was carried out using Illumina Sentrix® Human-1 Genotyping BeadChip (GUNDERSON et al., Nat. Genet., vol.37, p: 549-54, 2005) and Sentrix® HumanHap300 Genotyping BeadChips (STEEMERS et al., Nat. Methods., vol.3, p:31-3, 2006; ILLUMINA), according to manufacturers' instructions in a BeadLab with full automation.

All DNA samples were subjected to rigorous quality control to check for fragmentation and amplification. 20 µl of DNA at a concentration of 50 ng/μl was used for each array. DNA samples were tracked using a LIMS. The HumanHap300 Genotyping BeadChip was used with an Illumina LIMS while the Sentrix Human-1 Genotyping BeadChip was tracked through the ILLUMINA process by hand. Groups of 24 samples were batched. Five percent of the samples were selected from different batches, re-genotyped and the results compared to the original data. No sample discrepancies were detected. Raw data was analyzed using GTS Image and extracted for statistical analysis.

378 offspring were successfully genotyped with the Illumina Sentrix® HumanHap300 BeadChip (derived from HapMap Phase I), producing 317,497 SNPs with 34.7% average heterozygosity and 119,372,631 genotypes (99.5% call rate). 830 offspring and parents were genotyped using the Illumina Sentrix® Human-1 Genotyping BeadChip (concentrated on genes and surrounding sequences), producing an additional 91,293 SNPs with 36.0% average heterozygosity and 89,815,992 genotypes (99.0% call rate).

Tests of Hardy-Weinberg equilibrium were performed in cases and controls using the *genhwi* procedure (available at http://www.biostat-resources.com/stata/ and Stata™ version 9.2, and SNPs showing Hardy-Weinberg disequilibrium in controls ($\chi^2 > 25$) were excluded. As the data comprised a mixture of unrelated and related cases and controls, we used logistic regression models with robust sandwich estimation of the variance (WILLIAMS, Biometrics, vol.56, p: 645-6, 2000) as implemented in the Stata™ *logit* function to model clustering of sibling's genotypes. Simulations using the MRC-A family structures (data available on request) confirmed that this method appropriately controls the type I error. Heterogeneity of association between the two main strata (UK and Germany) was assessed by a weighted linear combination test using the results of an additive-effects-only regression analysis within each stratum. X-linked markers were analysed by fitting an additive-effects-only logit model which equates the risks of male hemizygotes with female homozygotes. The TRANSMIT program (CLAYTON, Am. J. Hum. Genet., vol.65, p: 1170-7, 1999) was used to analyse nuclear family data (including parental genotypes) using the sandwich variance estimation option to robustly incorporate information from multiple affected siblings. The overall statistical significance of the asthma GWA results was assessed by application of the QVALUE (available at http://faculty.washington.edu/~jstorey/qvalue/) software package (STOREY & TIBSHIRANI, Proc. Natl. Acad. Sci. USA, vol.100, p: 9440-5, 2003).

The test for Hardy-Weinberg equilibrium in 392 unrelated individuals identified 2.7% and 2.1% of SNPs deviating at the 0.01 level in the 100K and 300K panels respectively. We found only 0.412 Mendelian errors per SNP amongst the 102018 markers typed on all family members. These SNPs were included in analyses.

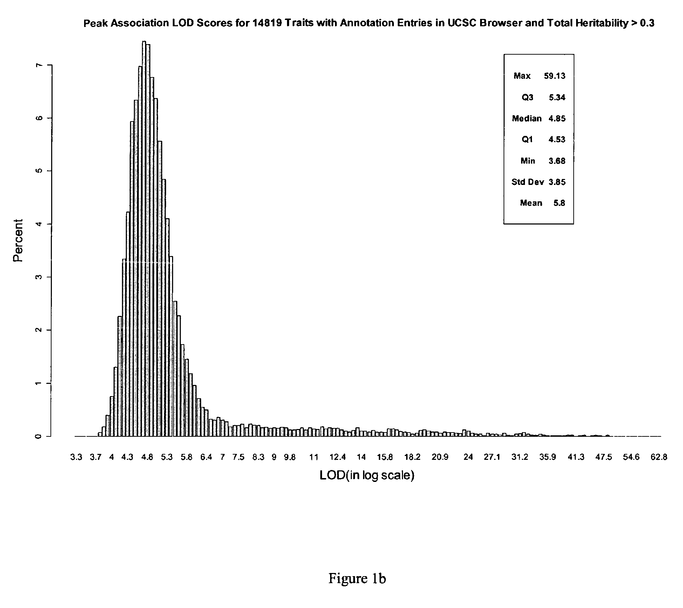

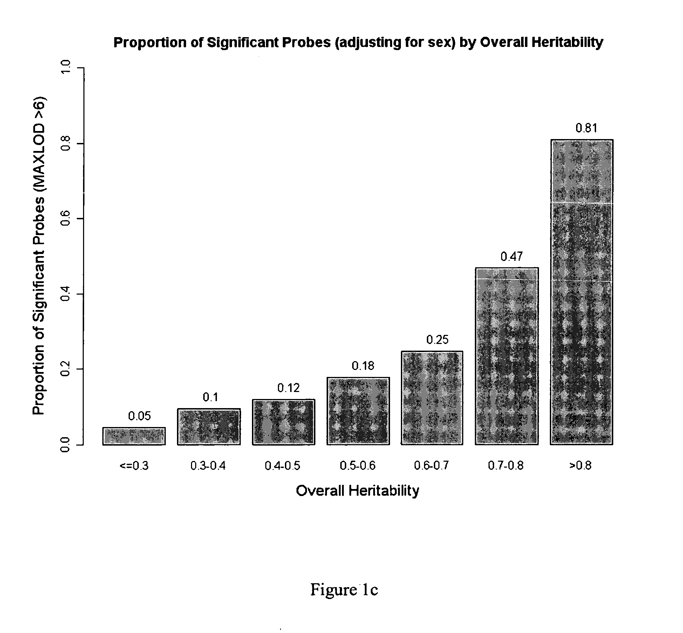

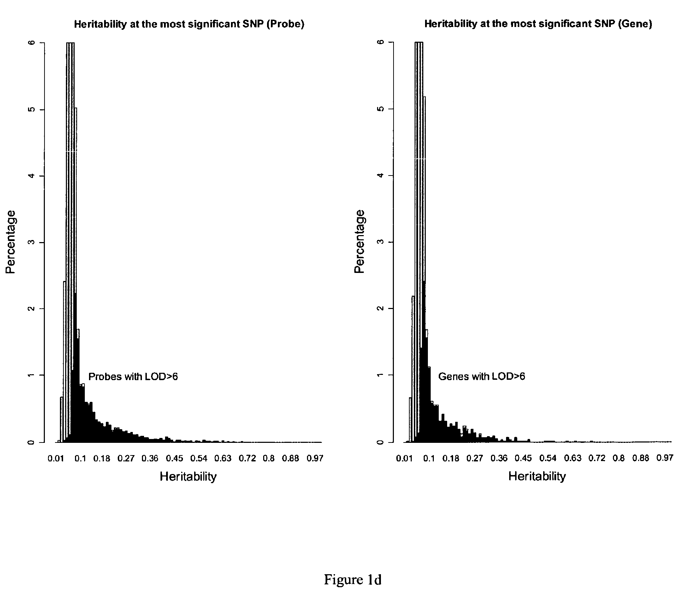

The results have shown that the Minor Allele Frequency (MAF) was predominately > 0.1 (Supplementary Figure 1). Our 408,273 successfully genotyped SNPs contained 372,821 SNPs from the 2,236,212 common SNPs (MAF>0.05) in HapMap database. The total coverage of HapMap at $R^2>0.8$ was 1,794,828 SNPs (80.3%), and for $R^2>0.5$ was 2,043,847 SNPs (91.4%). For the 317,149 SNPs tested in the 300K panel the coverage for $R^2>0.8$ was 1,762,736 SNPs (78.8%) and for $R^2>0.5$ was 2,032,597 SNPs (90.9%).

5) Genetic association to eQTL:

We applied the method of genomic control (DEVLIN et al., Theor. Popul. Biol., vol.60, p: 155-66, 2001) and derived a coefficient of 1.0099, indicating negligible population stratification.

Association analysis was applied with Merlin (FASTASSOC option; ABECASIS *et al.,* 2002, abovementioned). We estimated an additive effect for each SNP and tested its significance using a score test that adjusts for familiality and including sex in the model, which takes into account uncertainty in the inference of missing genotypes. In the absence of a positive genomic control test we did not adjust for stratification. We probabilistically inferred missing genotypes and adjusted for familiality, but not for linkage signal. A significance level of 0.05 and a Bonferroni correction applied to each expression trait individually led to a P-value threshold of $1.2 \times 10^{-7}$, corresponding to a lod score of 6.076. This equates to a false discovery rate of 0.049 for 408,273 SNPs tested for association with 15,084 transcripts (BENJAMINI & HOCHBERG, J. R. Statist. Soc. B., vol.57, p: 289-300, 1995).

The figure 1b shows the distribution of peak lod scores for association between traits with annotation entries in the UCSC browser and $H^2 > 0.3$, and SNP markers.

We found that the 14,819 traits with annotation entries in the UCSC browser and $H^2 > 0.3$ had a minimum lod score for association of 3.683, and a maximum of 59.128 (median 4.853, Q3 5.339) (Figure 1b). We estimated the threshold for genome wide significance to be a peak lod score > 6.076. We found the false discovery rate (FDR) for a lod score of 5.5 to be 0.152, for a lod of 6 to be 0.056, for a lod of 7.0 to be 0.0067, and for a lod of 8 to be 0.0008.

When the peak associated lod was >6, 78% of the most strongly associated SNPs were on the same chromosome as the transcripts and 89% of these SNPs were within the transcript. Only 22% of transcripts were associated with SNPs on different chromosomes. The median distance between a transcript and SNPs associated with its expression was approximately 9.2 kb.

The figure 1c shows the proportion of traits with peak LOD > 6 according to total trait heritability, wherein the results show that traits with higher total heritability were, on average, more mappable.

On average, we observed a higher proportion of peak LOD scores above our threshold (lod > 6.0) for traits

with higher $H^2$. For example, 81 % of traits with $H^2$ > 0.80 showed association to a SNP in our panels with lod > 6.0 (Figure 1c). This proportion decreased to only 5% for traits with a heritability of <30%. This may suggest that increases in sample size or selection for trait distribution may have more impact on the power of GWA studies than increases in marker density.

Five of our 50 most significant eQTL had been identified previously (*RPS26, IRF5, LRAP, CHI3L2* and *HSD17B12;* MORLEY *et al.,* 2004, abovementioned), showing eQTL mapping to be reproducible. The presence of numerous other highly significant eQTLs in our study is attributable to the larger sample size and more extensive exploration of the genome that we have undertaken, and creates a resource for the systematic mapping of complex disease genes.

For each trait, we examined the SNP showing strongest association.

The figure 1d shows the total heritability accounted for by the SNP showing strongest association with gene expression levels. On average, we observed larger effects when the peak associated SNP mapped in *cis* (red bars, within 100kb of target) of the probe or gene being evaluated than when it mapped in trans (blue bars). The right panel shows results after averaging all probes for each gene, as in Figure 1a. The % scale on the y axis is truncated at 6.0.

The mean $H^2$ attributable to this SNP was 0.066 (SD 0.029, max 0.707, sum 3629) compared to 0.203 for the overall $H^2$ of these traits (SD 0.170, max 1.0, sum 11104). This indicated that genotypes at a single SNP could explain 32.5% of the heritable component of these traits (Figure 1d). The $H^2$ attributable to a single SNP rose to 0.151 for the traits with a peak lod > 6.0, accounting for 29.3% of the $H^2$ for these traits.

We explored the unexplained heritability by testing for dominance and interaction effects amongst the 5824 genes with $H^2$ > 0.3 that could not be mapped (maxlod < 6) under the additive model. We identified 408 genes under a dominant model with P < $6.12x10^{-8}$ (Bonferroni correction for 2*408273 tests), which was less than the 634 genes observed in analysis with randomized genotypes. We tested for interactions amongst the top 100 SNPs for each of the 5824 genes, and found 363 genes had P < $6x10^{-8}$ (Bonferroni correction for 2*408273+10000 tests), compared to 210 genes observed in analysis with randomized genotypes. The results suggest that dominance had a minimal effect on gene transcription, and that interactions were minor.

Using a threshold $R^2$ > 0.8 in our genotype data we found that 79036 SNPs in the gene-targeted 100K panel were tagged by the HapMap derived 300K panel and 75098 SNPs in 300K panel could be tagged by the 100K panel. With both panels combined, we mapped 592 genes in *cis* with lod for association > 6. 209 genes mapped to the same SNP, 216 genes mapped at different *cis* SNPs in each panel, 64 genes mapped only using the 100K panel and 103 genes were mapped only using the 300K panel. Thus 10.8% of hits were specific to the 100K panel, 17.4% of hits were specific to the 300K and 71.8% of hits were identified with both panels. Consequently we typed only the 300K panel in the case-referent samples.

6) Gene Ontology:

We used Gene Ontology analyses to identify genes that were significantly enriched amongst highly heritable traits.

Transcripts and genes were matched to Gene Ontology (ASHBURNER et al., Nat. Genet., vol.25, p:25-9, 2000) categories using the HG-U133 Plus 2.0 annotation file (Dec 19th 2005) available from AFFYMETRIX NetAffx (available at http://www.affymetrix.com/support/technical/byproduct.affx?product=hg-ul 33-plus). We excluded GO annotations inferred from electronic annotation only, as these are considered less reliable (HARRIS et al., Nucleic Acids Res., vol. 32, p: D258-61, 2004). We investigated the average heritability of the transcripts assigned to each GO category using the statistic

$$Z_{H_i^2} = \frac{H_i^2 - \mu}{\sigma / \sqrt{n_i}}$$

where $H_i^2$ is the average heritability of the transcripts assigned to the i$^{th}$ GO category, $n_i$ is the number of transcripts in the i$^{th}$ category, $\mu$ and $\sigma$ are the overall mean and standard deviation of all heritability estimates, respectively.

The results are presented in the table 1 as follows:

Table 1: Gene Ontology of exceptionally heritable traits.

| GO-Biological Process | GO ID | $H^2$ | Z for $H^2$ |
|---|---|---|---|
| response to unfolded protein | 6986 | 0.38 | 9.03 |
| regulation of progression through cell cycle | 74 | 0.26 | 8.20 |
| RNA processing | 6396 | 0.30 | 7.85 |
| DNA repair | 6281 | 0.29 | 7.81 |
| protein folding | 6457 | 0.30 | 7.80 |
| immune response | 6955 | 0.26 | 7.62 |
| regulation of I-kappaB kinase/NF-kappaB cascade | 43123 | 0.28 | 6.84 |
| mitosis | 7067 | 0.30 | 5.82 |
| intracellular signaling cascade | 7242 | 0.26 | 5.72 |
| regulation of transcription | 45449 | 0.29 | 5.70 |
| regulation of viral genome replication | 45069 | 0.39 | 5.47 |
| protein biosynthesis | 6412 | 0.26 | 5.45 |
| vesicle-mediated transport | 16192 | 0.27 | 5.02 |
| cytokinesis | 910 | 0.32 | 4.94 |
| protein complex assembly | 6461 | 0.25 | 4.67 |
| DNA replication | 6260 | 0.27 | 4.61 |
| phosphoinositide-mediated signaling | 48015 5 | 0.32 | 4.59 |
| humoral immune response | 6959 | 0.30 | 4.47 |
| apoptosis | 6915 | 0.24 | 4.42 |

The analysis compared the mean total $H^2$ of transcripts in an individual GO category (GOID) with the mean total $H^2$ of all 54675 transcripts.

The results show that the most highly heritable GO biological process was "response to unfolded proteins". This group contained numerous chaperonins and heat shock proteins *(CRNN, 7 DNAJ* family members, *HERPUD1,* 16 *HSPA, B, C* or *D* family members, *SERPINH1, TOR1A* and *1B, TRA1* and *TXNDC4).*

The individual variation in response to unfolded proteins may represent an evolutionary response to cellular stress, and these genes could be candidates in the study of neurodegenerative diseases and aging processes.

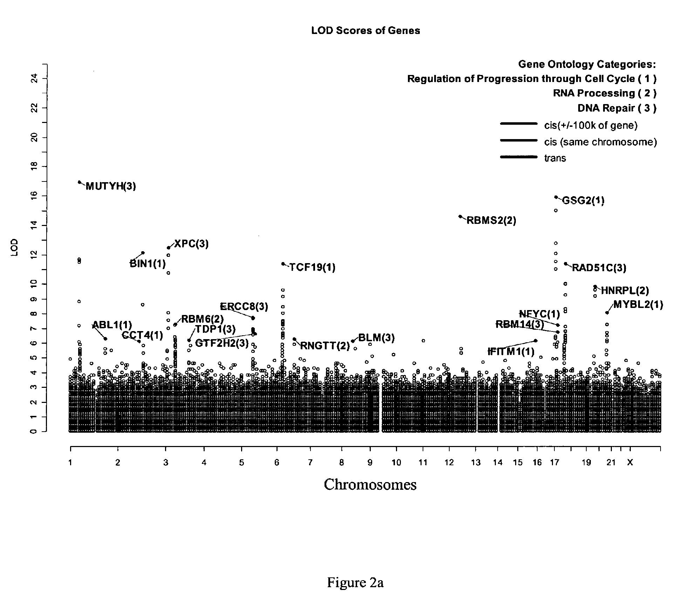

The figure 2a shows the mapping of genes with GO-BP descriptors for cell cycle, DNA repair and RNA processing, and overlays the results of genome wide association analysis for 20 genes annotated in the cell cycle, DNA repair and RNA processing GO Biological Process categories. The peak SNP for each trait is annotated with the gene name if the strength of association exceeds LOD > 6.0.

The results show that the genes regulating progression through cell cycle, RNA processing, and DNA repair were also exceptionally heritable (Figure 2a).

The evolutionary advantage of individual variation in these genes is unclear. These genes may be relevant candidates for the investigation of inherited susceptibility to cancer.

It has been shown previously that genes expressed in EBVL are enriched in GO categories of immune response (MONKS et al., Am. J. Hum. Genet., vol.75, p: 1094-105,2004).

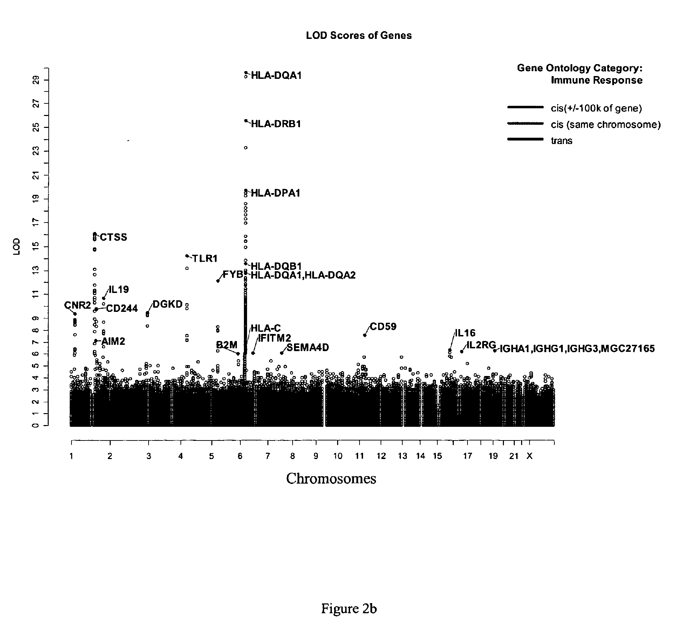

The mapping of 21 genes with GO-DP descriptors for immune responses is shown in figure 2b.

The results show the significant heritability that we observed to these traits (Figure 2b), which emphasizes their importance to inflammatory diseases. Several of these genes have an effect on asthma, including those of the *MHC, CHIA* (acid chitinase; ZHU et al., Science, vol.304, p: 1678-82, 2004; BIERBAUM et al., Am. J. Respir. Crit. Care. Med., vol.172, p:1505-9, 2005) and *IL16* (DE BIE et al., Clin Exp Allergy, vol.32, p: 1651-8, 2002; BURKART et al., J. Allergy. Clin. Immunol., vol117, p: 86-91, 2006). *IL2RG* is a key signalling component of many interleukin receptors, and *CD59* is ubiquitously expressed and protects cells against activated complement.

7) Asthma:

In order to test for association with the categorical phenotype of asthma, we further genotyped 728 physician-diagnosed asthmatic and 694 non-asthmatic children recruited from Germany and Austria, together with 437 non-asthmatic Caucasian UK controls.

Asthma cases from the Multicentre Asthma Genetics in Childhood Study (MAGICS) were diagnosed by a paediatric pulmonologist or allergologist based on clinical examination, case history and objective tests of lung function. Asthmatics (mean age 10.95 years) were recruited from 7 centres located in Germany and Austria (Wesel, Bochum Cologne, Freiberg, Munich, Feldkirch and Vienna), and as a reference, 800 German children (mean age 9.62 years) from Dresden (n=400) and Munich (n=400) were randomly drawn from all German children with DNA available in the cross sectional International Study of Asthma and Allergy in Childhood, phase II (WEILAND et al., Eur. Respir. J., vol. 24, p:406-12, 2004).

All study methods were approved by the appropriate ethics committees.

We used the Illumina Sentrix® HumanHap300 BeadChip as described previously, producing 317,447 SNPs and 452,361,975 genotypes (99.5% call rate).

We tested for association to asthma in the combined data set of 994 asthmatics and 1,224 non-asthmatics.

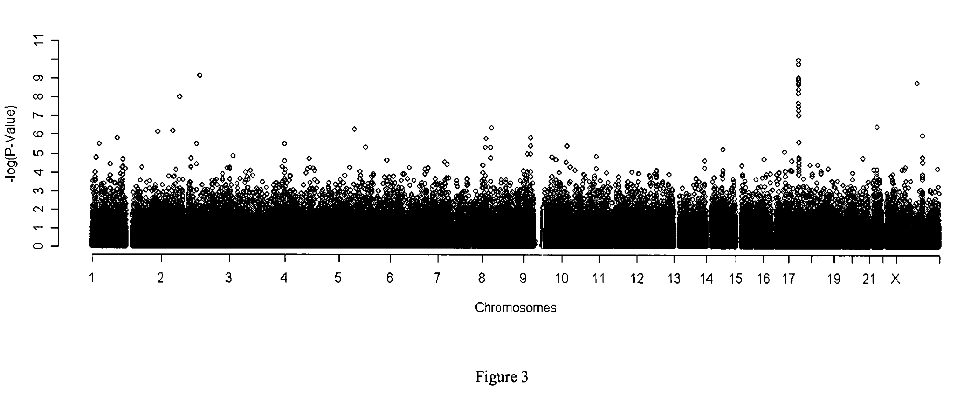

The figure 3 shows the genome-wide association of 317,447 SNPs and asthma in 994 asthmatic children and 1,244 non-asthmatic children.

The results show that numerous markers on chromosome 17q21 show association to asthma in the region of maximum association (figure 3). We calculated that a 1% FDR corresponded to a $P \leq 6.8 \times 10^{-7}$, and found 20 SNPs below this threshold. The 5% FDR threshold was $5.0 \times 10^{-6}$ (34 SNPs) and the 10% threshold was $1.1 \times 10^{-5}$ (37 SNPs), suggesting the presence of multiple susceptibility alleles for asthma.

Analysis for population stratification in the combined dataset found a genomic control parameter of 1.079, reflecting differences in allele frequencies within the European population. We controlled for admixture by modeling population stratification [13] with 102 randomly selected informative markers in HWE in a backward stepwise regression for the 34 SNPs showing association to asthma below the 5% FDR threshold. This had a minimal effect on association to any of these loci.

Potential associations below the 1% FDR included several markers that had only been typed in one of the panels or had exceptionally low minor allele frequencies. When these were excluded 16 SNPs remained and are presented in the following table 2:

Table 2: Markers that exceed the FDR 5% threshold for association with childhood asthma after adjustment for population stratification

| Marker | Chromosome | Location (pb) | Uncorrected P-value | Corrected P-value |
|---|---|---|---|---|
| rs10924993 | 1 | 238,526,408 | 7.E-07 | 3.E-06 |
| rs6716266 | 2 | 69,098,732 | 1.E-08 | 9.E-08 |
| rs8179521 | 2 | 127,867,394 | 3.E-06 | 8.E-07 |
| rs3791244 | 2 | 138,448,235 | 7.E-10 | 5.E-08 |
| rs4512342 | 8 | 32,727,416 | 4.E-07 | 2.E-09 |
| rs2666781 | 10 | 22,245,682 | 4.E-06 | 4.E-06 |
| rs907092 | 17 | 35,175, 785 | 1.E-09 | 8.E-07 |
| rs9303277 | 17 | 35,229,995 | 2.E-09 | 5.E-08 |
| rs11557467 | 17 | 35,282,160 | 8.E-10 | 3.E-08 |
| rs8067378 | 17 | 35,304,874 | 9.E-10 | 4.E-08 |
| rs2305480 | 17 | 35,315,722 | 3.E-09 | 5.E-07 |
| rs2290400 | 17 | 35,319,766 | 2.E-10 | 4.E-09 |
| rs7216389 | 17 | 35,323,475 | 9.E-11 | 3.E-09 |
| rs4795405 | 17 | 35,341,943 | 2.E-09 | 5.E-07 |
| rs2037986 | 21 | 28,398,348 | 4.E-07 | 6.E-07 |
| rs2311978 | I X | 75,705,247 | 2.E-09 | I 2.E-07 |

Strikingly, 7 of the 12 markers still below the 1% FDR threshold mapped to a 112 kb interval on chromosome 17q21.

Several other markers in this interval also showed strong evidence of association. The table 3a shows the results of association analysis for markers on chromosome 17q21 in the UK, German and combined panels used in the GWA study. Markers are either from the HumanHap300 set (origin=GWA) or supplementary markers of the region selected from dsSNP.

Table 3a

| Marker | Location | -Log10 (p-value) | | |
|---|---|---|---|---|
| | | UK | Germany | Combined |
| rs907092 | 35,175,785 | 4.97 | 4.66 | 8.92 |
| rs9911688 | 35,197,327 | 0.31 | 0.49 | 0.63 |
| rs9303277 | 35,229,995 | 4.40 | 5.02 | 8.79 |
| rs2060941 | 35,236,409 | 0.47 | 0.00 | 0.27 |
| rs9908694 | 35,251,298 | 0.28 | 0.64 | 0.76 |
| rs11557467 | 35,282,160 | 4.57 | 5.15 | 9.07 |
| rs8067378 | 35,304,874 | 4.34 | 5.31 | 9.03 |
| rs2123685 | 35,307,415 | 0.95 | 5.67 | 4.76 |
| rs8069176 | 35,310,723 | 3.61 | 6.42 | 9.42 |
| rs2305480 | 35,315,722 | 3.80 | 5.24 | 8.48 |
| rs2305479 | 35,315,743 | 5.07 | 6.62 | 11.05 |
| rs2290400 | 35,319,766 | 3.96 | 9.81 | 6.44 |
| rs4795400 | 35,320,546 | 3.56 | 4.60 | 7.60 |
| rs7216389 | 35,323,475 | 4.25 | 6.39 | 10.04 |
| rs9303281 | 35,327,572 | 4.59 | 7.81 | 11.74 |
| rs7219923 | 35,328,044 | 5.04 | 7.39 | 11.78 |
| rs3169572 | 35,330,938 | 0.52 | 0.84 | 1.12 |
| rs4378650 | 35,334,391 | 4.43 | 7.11 | 10.90 |
| rs8076131 | 35,334,438 | 3.80 | 6.53 | 9.72 |
| rs3744246 | 35,337,876 | 0.88 | 1.40 | 1.97 |
| rs4795402 | 35,338,911 | 1.52 | 2.10 | 3.22 |
| rs4795403 | 35,339,248 | 1.29 | 1.46 | 2.40 |
| rs4795404 | 35,339,317 | 1.13 | 1.27 | 2.07 |
| rs4795405 | 35,341,943 | 4.06 | 5.26 | 8.70 |
| rs4794820 | 35,342,870 | 3.90 | 5.56 | 8.87 |
| rs7207600 | 35,345,186 | 2.16 | 3.72 | 5.38 |
| rs8079416 | 35,346,239 | 2.42 | 6.49 | 8.23 |
| rs6503525 | 35,348,700 | 2.87 | 7.01 | 9.26 |
| rs8065126 | 35,352,561 | 2.36 | 4.40 | 6.23 |
| rs3893044 | 35,356,542 | 3.28 | 4.77 | 7.47 |
| rs4795408 | 35,361,153 | 2.11 | 6.08 | 7.53 |
| rs7209742 | 35,362,234 | 2.52 | 4.38 | 6.36 |

(continued)

| Marker | Location | -Log10 (p-value) | | |
|---|---|---|---|---|
| | | UK | Germany | Combined |
| rs8076474 | 35,364,760 | 2.52 | 4.45 | 6.43 |
| rs1007654 | 35,364,880 | 2.08 | 4.07 | 5.65 |
| rs1007655 | 35,364,945 | 2.40 | 4.01 | 5.90 |
| rs2313640 | 35,365,371 | 2.50 | 4.12 | 6.09 |
| rs7218742 | 35,367,887 | 2.59 | 4.16 | 6.22 |
| rs7218321 | 35,367,995 | 2.33 | 3.84 | 5.66 |
| rs7219080 | 35,368,042 | 1.79 | 4.35 | 5.60 |
| rs6503526 | 35,368,124 | 2.93 | 6.67 | 8.98 |
| rs6503527 | 35,368,245 | 2.43 | 3.83 | 5.75 |
| rs3902025 | 35,372,780 | 2.67 | 4.92 | 7.06 |
| rs3894194 | 35,375,519 | 1.92 | 6.63 | 7.72 |
| rs7212938 | 35,376,206 | 2.05 | 0.79 | 1.05 |
| rs3859192 | 35,382,174 | 1.98 | 7.30 | 6.00 |
| rs921651 | 35,387,448 | 0.25 | 1.27 | 1.25 |
| rs8066582 | 35,400,455 | 1.36 | 1.38 | 2.35 |
| rs8080546 | 35,400,492 | 0.27 | 1.59 | 1.52 |
| rs2071369 | 35,425,831 | 0.96 | 0.90 | 1.55 |
| rs2302776 | 35,431,675 | 1.60 | 3.04 | 4.19 |
| rs3934886 | 35,449,021 | 0.04 | 0.68 | 0.44 |
| rs11078936 | 35,451,440 | 1.92 | 2.91 | 4.38 |
| rs868150 | 35,466,885 | 1.53 | 1.65 | 2.78 |
| rs7502966 | 35,470,048 | 0.92 | 0.59 | 1.19 |
| rs1568400 | 35,474,634 | 0.25 | 0.04 | 0.10 |
| rs939348 | 35,485,379 | 0.59 | 0.28 | 0.64 |
| rs3744805 | 35,501,880 | 0.39 | 0.77 | 0.95 |
| rs2071427 | 35,508,018 | 0.30 | 0.17 | 0.34 |
| rs2269457 | 35,508,215 | 0.24 | 0.65 | 0.72 |
| rs2071570 | 35,510,616 | 0.63 | 0.23 | 0.61 |
| rs7211770 | 35,542,529 | 0.27 | 1.02 | 1.05 |
| rs2280400 | 35,602,653 | 0.05 | 0.55 | 0.33 |
| rs13706 | 35,710,677 | 0.15 | 1.27 | 0.65 |

The results identified significant SNP markers having a p value equal or greater than 6. The results further established that the patterns of association for the chromosome 17q21 markers were similar in both the UK family panel and the German case-referent panel. There was no evidence of heterogeneity of the association or of significant allele frequency differences in cases or controls from the UK and Germany.

We selected 27 markers from the dbSNP database that were within or adjacent to the strongly associated interval for genotyping. These exhibited similar patterns and strength association as the GWA markers (Table 3a).

Table 3b shows results of family based analysis (TRANSMIT) in 116 SNPs from the association region on chromosome 17q21 genotyped in parents and children of UK childhood asthma family.

Table 3b

| Marker | Location (bp) | -Log10(pvalue) |
|---|---|---|
| rs1989955 | 34,515,900 | 0.96 |
| rs16230 | 34, 521, 940 | 0.42 |
| rs498973 | 34,541,591 | 0.50 |
| rs16495 | 34,541,657 | 0.43 |
| rs645167 | 34,553,967 | 0.01 |
| rs563525 | 34,562,881 | 0.19 |
| rs16522 | 34, 570, 514 | 0.41 |
| rs544198 | 34,572,555 | 0.14 |
| rs657672 | 34,572,591 | 0.56 |
| rs657723 | 34,572,629 | 0.15 |
| rs16525 | 34,575,131 | 0.27 |
| rs3744353 | 34,587,769 | 078 |
| rs2240083 | 34,604,929 | 0.21 |
| rs521633 | 34,608,592 | 0.23 |
| rs16530 | 34, 610, 560 | 0.32 |
| rs11657409 | 34,621,682 | 0.22 |
| rs2338755 | 34,672,843 | 2.21 |
| rs8079590 | 34,679,727 | 2.12 |
| rs588193 | 34,693,965 | 2.12 |
| rs2302073 | 34,710,868 | 2.30 |
| rs2338800 | 34,751,675 | 2.12 |
| rs6503513 | 34,815,139 | 1.77 |
| rs9646419 | 34,850,711 | 2.54 |
| rs4795369 | 34,862,646 | 1.97 |
| rs11655550 | 34,863,649 | 3.12 |
| rs4390625 | 34,873,873 | 1.76 |
| rs12948906 | 34,898,380 | 1.89 |
| rs6503518 | 34,913,995 | 2.07 |
| rs7503705 | 34,923,230 | 2.08 |
| rs1619021 | 34,992,800 | 2.06 |
| rs9889354 | 35,011,493 | 0.05 |
| rs907094 | 35,043,897 | 0.83 |
| rs3764352 | 35,044,465 | 0.84 |
| rs881844 | 35,063,744 | 1.25 |
| rs1877031 | 35,067,606 | 1.19 |
| rs876493 | 35,078,071 | 1.10 |
| rs14050 | 35, 081, 598 | 2.52 |

(continued)

| Marker | Location (bp) | -Log10(pvalue) |
|---|---|---|
| rs2941503 | 35,082,271 | 2.67 |
| rs907087 | 35,082,313 | 2.52 |
| rs2517954 | 35,097,076 | 2.55 |
| rs2517955 | 35,097,207 | 2.00 |
| rs2517956 | 35,097,385 | 2.45 |
| rs1810132 | 35,119, 531 | 2.61 |
| rs907091 | 35,175,268 | 5.30 |
| rs907092 | 35,175,785 | 3.84 |
| rs10445308 | 35,191,573 | 3.96 |
| rs9911634 | 35,197,257 | 1.10 |
| rs10852936 | 35,285,240 | 3.62 |
| rs1054609 | 35,286,803 | 3.48 |
| rs8067378 | 35,304,874 | 5.51 |
| rs2123685 | 35,307,415 | 1.25 |
| rs8069176 | 35,310,723 | 3.98 |
| rs2305480 | 35,315,722 | 3.75 |
| rs2305479 | 35,315,743 | 5.32 |
| rs11078926 | 35,316,502 | 3.36 |
| rs1008723 | 35,319,793 | 4.65 |
| rs4795400 | 35,320,546 | 3.20 |
| rs7216389 | 35,323,475 | 5.27 |
| rs9303281 | 35,327,572 | 4.73 |
| rs7219923 | 35,328,044 | 5.01 |
| rs3169572 | 35,330,938 | 1.11 |
| rs4378650 | 35,334,391 | 4.67 |
| rs8076131 | 35,334,438 | 3.62 |
| rs3744246 | 35,337,876 | 1.58 |
| rs4795402 | 35,338,911 | 2.24 |
| rs4795403 | 35,339,248 | 1.47 |
| rs4795404 | 35,339,317 | 1.47 |
| rs4795405 | 35,341,943 | 3.30 |
| rs4794820 | 35,342,870 | 3.35 |
| rs7207600 | 35,345,186 | 1.56 |
| rs6503525 | 35,348,700 | 3.79 |
| rs8065126 | 35,352,561 | 2.03 |
| rs3893044 3 | 5,356,542 | 2.12 |
| rs4795408 | 35,361,153 | 3.33 |
| rs7209742 | 35,362,234 | 2.04 |

(continued)

| Marker | Location (bp) | -Log10(pvalue) |
|---|---|---|
| rs8076474 | 35,364,760 | 1.90 |
| rs1007654 | 35,364,880 | 1.73 |
| rs1007655 | 35,364,945 | 1.73 |
| rs2313640 | 35,365,371 | 1.90 |
| rs7218742 | 35,367,887 | 1.73 |
| rs7218321 | 35,367,995 | 1.47 |
| rs7219080 | 35,368,042 | 1.28 |
| rs6503526 | 35,368,124 | 3.87 |
| rs6503527 | 35,368,245 | 1.73 |
| rs3902025 | 35,372,780 | 1.76 |
| rs3894194 | 35,375,519 | 3.76 |
| rs7212938 | 35,376,206 | 3.11 |
| rs8077456 | 35,382,291 | 1.49 |
| rs3859188 | 35,396,504 | 0.00 |
| rs9915252 | 35,398,614 | 1.11 |
| rs2012 | 35,407,610 | 0.13 |
| rs2227319 | 35,424,371 | 0.77 |
| rs25645 | 35,426,669 | 1.23 |
| rs1042658 | 35,427,428 | 0.88 |
| rs1045929 | 35,428,952 | 1.04 |
| rs12309 | 35,428,988 | 1.45 |
| rs709591 | 35,429,087 | 1.00 |
| rs3213762 | 35,432,153 | 0.79 |
| rs2302775 | 35,436,370 | 0.17 |
| rs3935280 | 35,442,581 | 0.80 |
| rs868150 | 35,466,885 | 0.74 |
| rs7502966 | 35,470,048 | 0.18 |
| rs1879265 | 35,484,902 | 0.09 |
| rs3744805 | 35,501,880 | 0.20 |
| rs939347 | 35,510,219 | 0.49 |
| rs3744806 | 35,605,152 | 0.26 |
| rs4566234 | 35,667,875 | 0.00 |
| rs9916460 | 35,681,591 | 0.07 |
| rs9908257 | 35,681,648 | 0.16 |
| rs2077464 | 35,700,090 | 0.05 |
| rs13706 | 35,710,677 | 0.09 |
| rs2120200 | 35,713,901 | 0.09 |

SNPs from the region that we had typed in whole families in the MRC-A panel showed significant associations in a family based test, with SNP markers having a p value equal or greater than 2.5.

A supplemental analysis reveals an association with asthma of *ORMDL3* gene (chromosome 17q21), which is the third member of a novel class of genes of unknown function that encode transmembrane proteins anchored in the endoplasmic reticulum (ER; HJELMQVIST *et al.,* 2002, abovementioned).

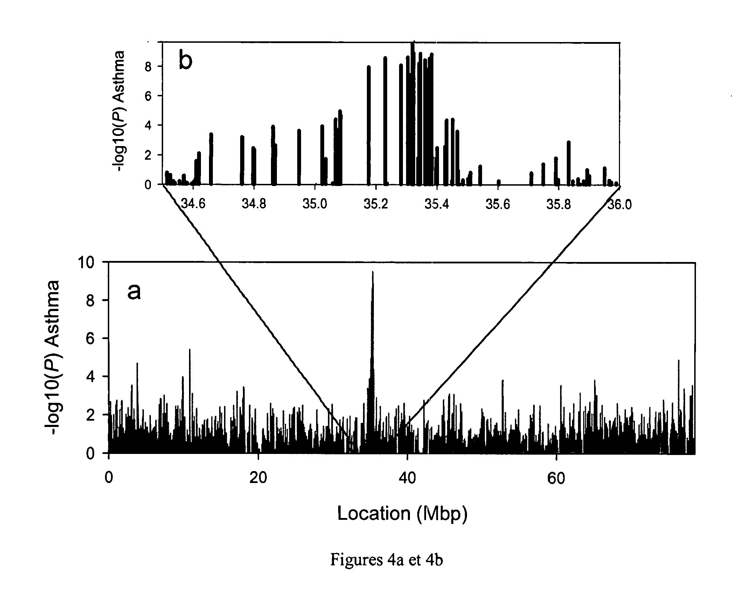

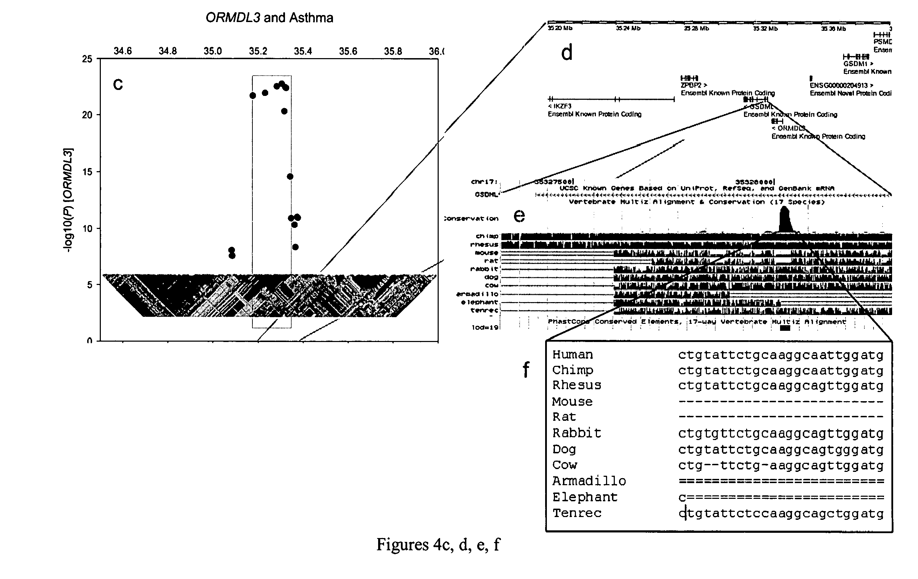

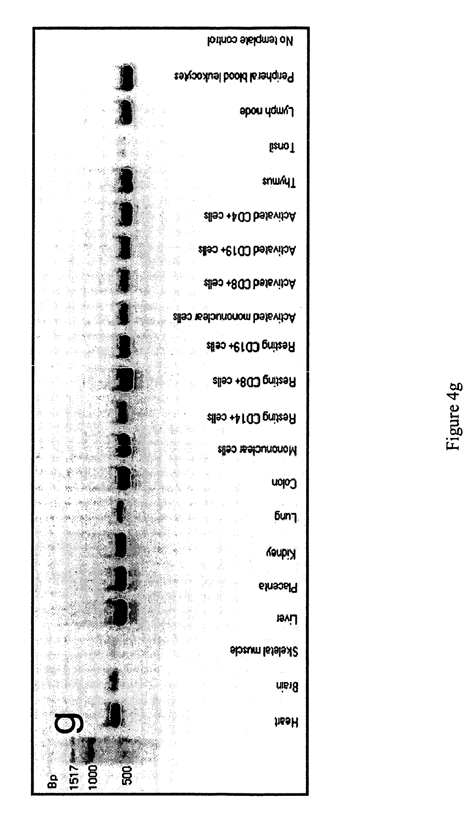

The figure 4 shows the association to asthma and increased transcript abundances of *ORMDL3* on chromosome 17q21. a) Mapping of association to asthma on chromosome 17, b) Detail of association to SNPs on chromosome 17q21, c) association to ORMDL3 transcript abundance with the same markers. A GOLD plot 30 of linkage disequilibrium (LD) between markers is also shown, with red indicating high LD and blue low. The central island of LD, which contains maximum association to ORMDL3 and asthma, is contained within the grey rectangle, d) genes contained within the associated interval e) homology plot from region of maximum association f) sequence homology from intron I of GSDML, g) RT-PCR (34 cycles) of ORMDL3 cDNA from representative tissues (Clontech Ltd).

We examined multiple tissue cDNA panels by RT-PCR, and found that ORMDL3 to be expressed in many tissues, particularly liver and peripheral blood lymphocytes (Figure 4g).

The table 4a shows the association of asthma and *ORMDL3* to chromosome 17q21.1 in combined panels.

Table 4a

| Marker | Location (Mbp) | -log10(P) [*ORMDL3*] Family | allele_1 | allele_2 | freq. allele 1 asthmatic | freq. allele 2 asthmatic |
|---|---|---|---|---|---|---|
| *rs9303277* | 35.230 | 21.9 | C | T | 0,58 | 0,42 |
| *rs11557467* | 35.282 | 22.5 | G | T | 0,58 | 0,42 |
| *rs8067378* | 35.305 | 22.7 | A | G | 0,58 | 0,42 |
| *rs2290400* | 35.320 | 22.4 | A | G | 0,59 | 0,41 |
| *rs7216389* | 35.323 | 22.4 | T | C | 0,58 | 0,42 |
| *rs4795405* | 35.342 | 14.6 | C | T | 0,63 | 0,37 |
| *rs8079416* | 35.346 | 10.9 | C | T | 0,53 | 0,47 |
| *rs4795408* | 35.361 | 10.3 | A | G | 0,52 | 0,48 |
| *rs3894194* | 35.376 | 11.0 | A | C | 0,53 | 0,47 |
| *rs3859192* | 35.382 | 3.5 | T | C | 0,51 | 0,49 |

SNPs are from the Illumina 300K panel. The results are calculated with logistic regression models. Association to *ORMDL3* transcript abundance is shown for comparison and is based on the family panel only.

Table 4b: Transmission disequilibrium tests of association of asthma to chromosome 17q21.1 in family panels.

TRANSMIT

| Marker | Location (Mbp) | Informative Transmissions | $\chi^2$ | -log10(*P*) | GRR | 95% | CI | -log10(P) [ORMDL3] |
|---|---|---|---|---|---|---|---|---|
| *rs907091* | 35.175 | 245 | 20.8 | 5.3 | 1.82 | 1.41 | 2.40 | 24.1 |
| *rs907092* | 35.175 | 232 | 14.4 | 3.8 | 1.66 | 1.28 | 2.20 | 21.7 |
| *rs10445308* | 35.191 | 275 | 15.0 | 4.0 | 1.61 | 1.27 | 2.07 | 21.4 |
| *rs2305480* | 35.315 | 246 | 14.0 | 3.7 | 1.63 | 1.27 | 2.13 | 20.3 |
| *rs1008723* | 35.320 | 245 | 18.0 | 4.6 | 1.74 | 1.35 | 2.29 | 23.7 |
| *rs7216389* | 35.323 | 225 | 20.7 | 5.3 | 1.87 | 1.44 | 2.50 | 22.4 |
| *rs9303281* | 35.327 | 246 | 18.2 | 4.7 | 1.75 | 1.36 | 2.29 | 25.0 |
| *rs 7219923* | 35.330 | 250 | 17.2 | 4.5 | 1.71 | 1.33 | 2.23 | 25.2 |
| *rs3894194* | 35.375 | 246 | 14.1 | 3.8 | 1.63 | 1.27 | 2.13 | 8.5 |

SNPs are from the Illumina 100K panel. Results of association to asthma are calculated using TRANSMIT. Association to *ORMDL3* transcript abundance is shown for comparison, and was calculated using MERLIN.

The results establish that 13 of the SNPs below the 5% FDR mapped to a 206 kb interval on chromosome 17 (Table 4a, Figure 4 and Figure 5), with the most strongly associated SNP was *rs7216389 (P =* 9.1 x 10[-11]). In fact, the T allele at *rs 7216389* has a frequency 62% amongst asthmatics compared to 52% in non-asthmatics. Association to chromosome 17 markers was present in the family and the case-referent panel, and was highly significant in both case/control and family-based tests of association (Table 4b).

A forward stepwise regression with all markers in the 34.5 to 36 Mb interval on chromosome 17 identified 3 SNPs (*rs7216389, rs11650680* and *rs3859192,*) showing independent statistical effects on asthma (global *P* = 7.1 x $10^{-13}$). This finding was consistent with the presence of more than one functional SNP, or the presence of untyped functional SNPs in incomplete LD with the typed markers.

We then examined the locus with our eQTL data, and found that transcripts in the *ORMDL3* gene were strongly ($P < 10^{-22}$) and consistently positively associated to the same SNPs as asthma (Figure 4) (correlation between asthma and *ORMDL3 P* values = 0.67, P = 0.004). No other transcripts from the region showed significant relationship to the disease-associated markers, indicating that the same polymorphisms that are associated with *ORMDL3* transcript levels are also associated with asthma susceptibility.

Finally, the SNPs showing the strongest association to asthma and *ORMDL3* transcript abundances are contained within an island of linkage disequilibrium between 35.2 and 35.4 Mbp on chromosome 17q21 (Figure 4b and 4c).

REFERENCES

1. Ober, C. & Hoffjan, S. Asthma genetics 2006: the long and winding road to gene discovery. Genes Immun 7, 95-100 (2006).

2. Cookson, W. & Moffatt, M. Making sense of asthma genes. N Engl J Med 351, 1794-6 (2004).

3. Schadt, E. E. et al. Genetics of gene expression surveyed in maize, mouse and man. Nature 422, 297-302 (2003).

4. Morley, M. et al. Genetic analysis of genome-wide variation in human gene expression. Nature 430, 743-7 (2004).

5. Cheung, V. G. et al. Natural variation in human gene expression assessed in lymphoblastoid cells. Nat Genet 33, 422-5 (2003).

6. Abecasis, G., Cardon., L. & Cookson, W. Selection strategies for disequilibrium mapping of quantitative traits in nuclear families. Am J Hum Genet 65, A245 (1999).

7. Devlin, B., Roeder, K. & Wasserman, L. Genomic control, a new approach to genetic-based association studies. Theor Popul Biol 60, 155-66 (2001).

8. Monks, S. A. et al. Genetic inheritance of gene expression in human cell lines. Am J Hum Genet 75, 1094-105 (2004).

9. Zhu, Z. et al. Acidic mammalian chitinase in asthmatic Th2 inflammation and IL-13 pathway activation. Science 304, 1678-82 (2004).

10. Bierbaum, S. et al. Polymorphisms and haplotypes of acid mammalian chitinase are associated with bronchial asthma. Am J Respir Crit Care Med 172, 1505-9 (2005).

11. De Bie, J. J. et al. Exogenous interleukin-16 inhibits antigen-induced airway hyper-reactivity, eosinophilia and Th2-type cytokine production in mice. Clin Exp Allergy 32, 1651-8 (2002).

12. Burkart, K. M. et al. Association of asthma with a functional promoter polymorphism in the IL16 gene. J Allergy Clin Immunol 117, 86-91 (2006).

13. Setakis, E., Stirnadel, H. & Balding, D. J. Logistic regression protects against population structure in genetic association studies. Genome Res 16, 290-6 (2006).

14. Hjelmqvist, L. et al. ORMDL proteins are a conserved new family of endoplasmic reticulum membrane proteins. Genome Biol 3, RESEARCH0027 (2002).

15. Standards for the diagnosis and care of patients with chronic obstructive pulmonary disease (COPD) and asthma.

This official statement of the American Thoracic Society was adopted by the ATS Board of Directors, November 1986. Am Rev Respir Dis 136, 225-44 (1987).

16. British guideline on the management of asthma. Thorax 58 Suppl 1, i1-94 (2003).

17. Weiland, S. K. et al. Phase II of the International Study of Asthma and Allergies in Childhood (ISAAC II): rationale and methods. Eur Respir J 24, 406-12 (2004).

18. Gunderson, K. L., Steemers, F. J., Lee, G., Mendoza, L. G. & Chee, M. S. A genome-wide scalable SNP genotyping assay using microarray technology. Nat Genet 37, 549-54 (2005).

19. Steemers, F. J. et al. Whole-genome genotyping with the single-base extension assay. Nat Methods 3, 31-3 (2006).

20. Irizarry, R. A. et al. Exploration, normalization, and summaries of high density oligonucleotide array probe level data. Biostatistics 4, 249-64 (2003).

21. Bolstad, B. M., Irizarry, R. A., Astrand, M. & Speed, T. P. A comparison of normalization methods for high density oligonucleotide array data based on variance and bias. Bioinformatics 19, 185-93 (2003).

22. Abecasis, G. R., Cherny, S. S., Cookson, W. O. & Cardon, L. R. Merlin--rapid analysis of dense genetic maps using sparse gene flow trees. Nat Genet 30, 97-101 (2002).

23. Sham, P. C., Purcell, S., Cherny, S. S. & Abecasis, G. R. Powerful regression-based quantitative-trait linkage analysis of general pedigrees. Am J Hum Genet 71, 238-53 (2002).

24. Benjamini, Y. & Hochberg, Y. Controlling the false discovery rate: a practical and powerful approach to multiple testing. J. R. Statist. Soc. B. 57, 289-300 (1995).

25. Williams, R. L. A note on robust variance estimation for cluster-correlated data. Biometrics 56, 645-6 (2000).

26. Clayton, D. A generalization of the transmission/disequilibrium test for uncertain-haplotype transmission. Am J Hum Genet 65, 1170-7 (1999).

27. Storey, J. D. & Tibshirani, R. Statistical significance for genomewide studies. Proc Natl Acad Sci U S A 100, 9440-5 (2003).

28. Ashburner, M. et al. Gene ontology: tool for the unification of biology. The Gene Ontology Consortium. Nat Genet 25, 25-9 (2000).

29. Harris, M. A. et al. The Gene Ontology (GO) database and informatics resource. Nucleic Acids Res 32, D258-61 (2004).

30. Abecasis, G. R. & Cookson, W. O. GOLD--graphical overview of linkage disequilibrium. Bioinformatics 16, 182-3 (2000).

**Claims**

1. A method of testing a subject thought to have or be predisposed to having asthma, allergie, atopic disease or atopic sensitization, which comprises the step of analyzing a biological sample from said subject for:

   (i) detecting the presence of a mutation associated with the over-expression of a gene of the ORM1-like gene (ORMDL) family, and/or
   (ii) analyzing the expression of a gene of the ORMDL family.

2. The method according to claim 1, wherein said subject is thought to have or be predisposed to having asthma.

3. The method according to any one of claims 1 or 2, wherein said subject is a mammal, preferably a human.

4. The method according to any one of claims 1 to 3, wherein the gene of the ORMDL family is the ORMDL3 gene.

5. The method according to any one of claims 1 to 4, wherein said mutation (s) associated with the over-expression of a gene of the ORM1-like gene (ORMDL) family corresponds to a single nucleotide polymorphism (SNP).

6. The method according to any one of claims 1 to 5, wherein said mutation(s) associated with the over-expression of a gene of the ORM1-like gene (ORMDL) family has the same chromosomal location as said gene.

7. The method according to any one of claims 1 to 6, wherein said mutation is a single nucleotide polymorphism associated with the over-expression of the ORMDL3 gene in a human, and wherein said mutation is located on chromosome 17q21 and is selected from the group comprising *rs9303277* (nucleotide N at position 128 of SEQ ID NO:3, wherein allele C is associated to asthma), *rs11557467* (nucleotide N at position 128 of SEQ ID NO:4, wherein allele G is associated to asthma), *rs8067378* (nucleotide N at position 128 of SEQ ID NO:5, wherein allele A is associated to asthma), *rs2290400* (nucleotide N at position 128 of SEQ ID NO:6, wherein allele A is associated to asthma), *rs7216389* (nucleotide N at position 128 of SEQ ID NO:7, wherein allele T is associated to asthma), *rs4795405* (nucleotide N at position 128 of SEQ ID NO:8, wherein allele C is associated to asthma), *rs8079416* (nucleotide N at position 128 of SEQ ID NO:9, wherein allele C is associated to asthma), *rs4795408* (nucleotide N at position 128 of SEQ ID NO:10, wherein allele A is associated to asthma), *rs3894194* (nucleotide N at position 128 of SEQ ID NO:11, wherein allele T is associated to asthma), *rs3859192* (nucleotide N at position 128 of SEQ ID NO:12, wherein allele T is associated to asthma), rs9646419 (nucleotide N at position 128 of SEQ ID NO:13, wherein allele A is associated to asthma), rs14050 (nucleotide N at position 128 of SEQ ID NO:14, wherein allele C is associated to asthma), rs2941503 (nucleotide N at position 128 of SEQ ID NO:15, wherein allele A is associated to asthma), rs907087 (nucleotide N at position 128 of SEQ ID NO:16, wherein allele G is associated to asthma), rs2517954 (nucleotide N at position 128 of SEQ ID NO:17, wherein allele T is associated to asthma), rs1810132 (nucleotide N at position 128 of SEQ ID NO:18, wherein allele C is associated to asthma), rs907091 (nucleotide N at position 128 of SEQ ID NO:19, wherein allele T is associated to asthma), rs907092 (nucleotide N at position 128 of SEQ ID NO:20, wherein allele G is associated to asthma), rs10445308 (nucleotide N at position 128 of SEQ ID NO:21, wherein allele C is associated to asthma), rs10852936 (nucleotide N at position 128 of SEQ ID NO:22, wherein allele C is associated to asthma), rs1054609 (nucleotide N at position 128 of SEQ ID NO:23, wherein allele A is associated to asthma), rs8067378 (nucleotide N at position 128 of SEQ ID NO:24, wherein allele A is associated to asthma), rs2123685 (nucleotide N at position 128 of SEQ ID NO:25, wherein allele C is associated to asthma), rs8069176 (nucleotide N at position 128 of SEQ ID NO:26, wherein allele G is associated to asthma), rs2305480 (nucleotide N at position 128 of SEQ ID NO:27, wherein allele G is associated to asthma), rs2305479 (nucleotide N at position 128 of SEQ ID NO: 14, wherein allele C is associated to asthma), rs11078926 (nucleotide N at position 128 of SEQ ID NO:29, wherein allele G is associated to asthma), rs1008723 (nucleotide N at position 128 of SEQ ID NO:30, wherein allele G is associated to asthma), rs4795400 (nucleotide N at position 128 of SEQ ID NO:31, wherein allele C is associated to asthma), rs7216389 (nucleotide N at position 128 of SEQ ID NO:32, wherein allele T is associated to asthma), rs9303281 (nucleotide N at position 128 of SEQ ID NO:33, wherein allele A is associated to asthma), rs7219923 (nucleotide N at position 128 of SEQ ID NO:34, wherein allele T is associated to asthma), rs3169572 (nucleotide N at position 128 of SEQ ID NO:35, wherein allele A is associated to asthma), rs4378650 (nucleotide N at position 128 of SEQ ID NO:36, wherein allele G is associated to asthma), rs8076131 (nucleotide N at position 128 of SEQ ID NO:37, wherein allele A is associated to asthma), rs3744246 (nucleotide N at position 128 of SEQ ID NO:38, wherein allele C is associated to asthma), rs4795402 (nucleotide N at position 128 of SEQ ID NO:39, wherein allele C is associated to asthma), rs4795403 (nucleotide N at position 128 of SEQ ID NO:40, wherein allele C is associated to asthma), rs4795404 (nucleotide N at position 128 of SEQ ID NO:41, wherein allele C is associated to asthma), rs4795405 (nucleotide N at position 128 of SEQ ID NO:42, wherein allele C is associated to asthma), rs4794820 (nucleotide N at position 128 of SEQ ID NO:43, wherein allele G is associated to asthma), rs7207600 (nucleotide N at position 128 of SEQ ID NO:44, wherein allele A is associated to asthma), rs6503525 (nucleotide N at position 128 of SEQ ID NO:45, wherein allele C is associated to asthma), rs8065126 (nucleotide N at position 128 of SEQ ID NO:46, wherein allele C is associated to asthma), rs3893044 (nucleotide N at position 128 of SEQ ID NO:47, wherein allele C is associated to asthma), rs4795408 (nucleotide N at position 128 of SEQ ID NO:48, wherein allele A is associated to asthma), rs7209742 (nucleotide N at position 128 of SEQ ID NO:49, wherein allele G is associated to asthma), rs8076474 (nucleotide N at position 128 of SEQ ID NO:50, wherein allele C is associated to asthma), rs1007654 (nucleotide N at position 128 of SEQ ID NO:51, wherein allele G is associated to asthma), rs1007655 (nucleotide N at position 128 of SEQ ID NO:52, wherein allele A is associated to asthma), rs2313640 (nucleotide N at position 128 of SEQ ID NO:53, wherein allele T is associated to asthma), rs7218742 (nucleotide N at position 128 of SEQ ID NO:54, wherein allele G is associated to asthma), rs7218321 (nucleotide N at position 128 of SEQ ID NO:55, wherein allele T is associated to asthma), rs7219080 (nucleotide N at position 128 of SEQ ID NO:56, wherein allele C is associated to asthma), rs6503526 (nucleotide N at position 128 of SEQ ID NO:57, wherein allele T is associated to asthma), rs6503527 (nucleotide N at position 128 of SEQ ID NO:58, wherein allele A is associated to asthma), rs3894194 (nucleotide N at position 128 of SEQ ID NO:59, wherein allele A is associated to asthma), rs7212938 (nucleotide N at position 128 of SEQ ID NO:60, wherein allele T is associated to asthma), rs2305479 (nucleotide N at position 128 of SEQ ID NO:61, wherein allele C is associated to asthma), rs2305480 (nucleotide N at position 128 of SEQ ID NO:62, wherein allele G is associated to asthma), and rs2941503 (nucleotide N at position 128 of SEQ ID NO:63, wherein allele A is associated to asthma).

8. The method according to any one of claims 1 to 4, wherein the step (ii) of analyzing the expression of a gene of the ORMDL family is assessed by detecting expression of a transcribed nucleic acid or translated protein.

9. The method according to claim 8, wherein said method comprises the step of comparing the level of expression of a gene of the ORMDL family in said biological sample with the normal expression level of said gene in a control.

10. A use, for treating and/or preventing asthma, allergie, atopic disease or atopic sensitization in a subject, of a compound which specifically inhibits the expression of a gene of the ORMDL family.

11. The use according to claim 10, wherein said use is for treating and/or preventing asthma in a subject.

12. The use according to claim 10, wherein said compound specifically inhibiting the expression a gene of the ORMDL family is an oligonucleotide, which is selected in the group comprising anti-sense RNA and DNA molecules, ribozymes, siRNAs and aptamers.

13. The use according to claim 11, wherein said oligonucleotide is a siRNA, which is selected in the group comprising SEQ ID NO: 64 (CUAAGUACGACCAGAUCCA), SEQ ID O: 65 (AAGGCAUGUGCUGCAACAC), SEQ ID NO: 66 (AGAAGAAGCCUCUGGACAC), SEQ ID NO: 67 (GUAGCCAACUUGGAGUAGC), SEQ ID NO: 68 (UCAAUAAGUACUGAGAGUG), SEQ ID NO: 69 (UAAGUACUGAGAGUGCAGC), and SEQ ID NO: 70 (AGUUCUUGACCAUCACACC).

14. An *in vitro* method of selecting a compound, which can be useful for treating asthma, allergie, atopic disease or atopic sensitization, **characterized in that** said method comprises the steps of:

    a) obtaining a cell expressing a gene of the ORMDL family,
    b) contacting said cell with at least one compound,
    c) comparing the expression of the gene of the ORMDL family in the cell between the steps a) and b), and
    d) selecting the compound, which induces a lower level of expression of the gene of the ORMDL family in the cell contacted to that compound.

15. The method according to claim 14, wherein said method is for selecting a compound, which can be useful for treating asthma.

16. The method according to claim 14, wherein said gene of the ORMDL family corresponds to ORMDL3.

Figure 1a

EP 2 006 687 A1

Peak Association LOD Scores for 14819 Traits with Annotation Entries in UCSC Browser and Total Heritability > 0.3

| Max | 59.13 |
| Q3 | 5.34 |
| Median | 4.85 |
| Q1 | 4.53 |
| Min | 3.68 |
| Std Dev | 3.85 |
| Mean | 5.8 |

Figure 1b

**Proportion of Significant Probes (adjusting for sex) by Overall Heritability**

Figure 1c

Figure 1d

Figure 2a

Figure 2b

Figure 3

57

Figures 4a et 4b

Figures 4c, d, e, f

EP 2 006 687 A1

Figure 4g

**European Patent Office**

**PARTIAL EUROPEAN SEARCH REPORT**

which under Rule 63 of the European Patent Convention shall be considered, for the purposes of subsequent proceedings, as the European search report

Application Number

EP 07 30 1135

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 98/33817 A (INCYTE PHARMA INC [US]; HILLMAN JENNIFER L [US]; GOLI SURYA K [US]) 6 August 1998 (1998-08-06) * claim 1; figures 1A/B; lines 29-30 on page 9; from line 29 on page 20 to line 8 on page 21; the first full paragraph on page 30 * ----- | 1-12, 14-16 | INV. G01N33/68 ADD. A61P11/06 A61P37/08 |
| X | WO 03/073990 A (CHILDRENS HOSP MEDICAL CENTER [US]; ROTHENBERG MARC [US]; ZIMMERMANN N) 12 September 2003 (2003-09-12) | 1-3, 5-12,14, 15 | |
| Y | * claims 6,10,22,27,32,36,38 * ----- | 4,16 | |
| X | WO 00/70047 A (INCYTE GENOMICS INC [US]; YUE HENRY [US]; TANG Y TOM [US]; LAL PREETI) 23 November 2000 (2000-11-23) * page 49, line 14 - page 50, line 13; claim 1; sequence 22 * ----- | 1-12 | |
| | -/-- | | |

TECHNICAL FIELDS SEARCHED (IPC)

G01N

## INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

see sheet C

| Place of search | Date of completion of the search | Examiner |
|---|---|---|
| Munich | 27 November 2007 | Fausti, Simone |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04E07)

**European Patent Office**

**PARTIAL EUROPEAN SEARCH REPORT**

Application Number

EP 07 30 1135

| DOCUMENTS CONSIDERED TO BE RELEVANT | | | CLASSIFICATION OF THE APPLICATION (IPC) |
|---|---|---|---|
| Category | Citation of document with indication, where appropriate, of relevant passages | Relevant to claim | |
| X | WO 02/059260 A (HYSEQ INC [US]; TANG Y TOM [US]; GOODRICH RYLE W [US]; LIU CHENGHUA [U) 1 August 2002 (2002-08-01)<br>* page 51, line 15 - line 22; claims 1,20,27,28; sequence 353 *<br>----- | 14-16 | |
| Y | FAN CHAOHONG: "Orosomucoid types in allergic contact dermatitis"<br>HUMAN HEREDITY, KARGER, BASEL, CH, vol. 45, no. 2, 1995, pages 117-120, XP009092704<br>ISSN: 0001-5652<br>* abstract *<br>----- | 4,16 | |

TECHNICAL FIELDS SEARCHED (IPC)

EPO FORM 1503 03.82 (P04C10)

European Patent
Office

**INCOMPLETE SEARCH
SHEET C**

Application Number

EP 07 30 1135

Although claims 10-13 are directed to a method of treatment of the human/animal body (Article 53(c) EPC), the search has been carried out and based on the alleged effects of the compound/composition.

-----

**ANNEX TO THE EUROPEAN SEARCH REPORT
ON EUROPEAN PATENT APPLICATION NO.**

EP 07 30 1135

This annex lists the patent family members relating to the patent documents cited in the above-mentioned European search report.
The members are as contained in the European Patent Office EDP file on
The European Patent Office is in no way liable for these particulars which are merely given for the purpose of information.

27-11-2007

| Patent document cited in search report | | Publication date | Patent family member(s) | | Publication date |
|---|---|---|---|---|---|
| WO 9833817 | A | 06-08-1998 | AU | 5827498 A | 25-08-1998 |
| | | | US | 5863764 A | 26-01-1999 |
| WO 03073990 | A | 12-09-2003 | AU | 2003213633 A1 | 16-09-2003 |
| | | | CA | 2477400 A1 | 12-09-2003 |
| | | | CN | 1692161 A | 02-11-2005 |
| | | | EP | 1527196 A2 | 04-05-2005 |
| | | | JP | 2005532997 T | 04-11-2005 |
| | | | MX | PA04008286 A | 27-07-2005 |
| WO 0070047 | A | 23-11-2000 | AU | 5134600 A | 05-12-2000 |
| | | | CA | 2373191 A1 | 23-11-2000 |
| | | | EP | 1179065 A2 | 13-02-2002 |
| | | | JP | 2002543839 T | 24-12-2002 |
| WO 02059260 | A | 01-08-2002 | CA | 2429343 A1 | 01-08-2002 |
| | | | EP | 1341804 A2 | 10-09-2003 |
| | | | US | 2004137434 A1 | 15-07-2004 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 4683202 A
- US 5854033 A
- WO 0244321 A
- US 5270163 A

### Non-patent literature cited in the description

- **OBER ; HOFFJAN.** *Genes Immun.,* 2006, vol. 7, 95-100
- **COOKSON ; MOFFATT.** *N. Engl. J. Med.,* 2004, vol. 351, 1794-6
- **SCHADT et al.** *Nature,* 2003, vol. 422, 297-302
- **MORLEY et al.** *Nature,* 2004, vol. 430, 743-7
- **CHEUNG et al.** *Nat. Genet.,* 2003, vol. 33, 422-5
- **HJELMQVIST et al.** *Genome biology,* 2002, vol. 3 (6
- **BARANY.** *Proc. Natl. Acad. Sci. USA,* 1991, vol. 88, 189-193
- **GUATELLI et al.** *Proc. Natl. Acad. Sci. USA,* 1990, vol. 87, 1874-1878
- **KWOH et al.** *Proc. Natl. Acad. Sci. USA,* 1989, vol. 86, 1173-1177
- **LIZARDI et al.** *Biol. Technology,* 1988, vol. 6, 1197
- **KOHLER ; MILSTEIN.** *Nature,* 1975, vol. 256, 495-497
- **KOZBOR et al.** *Immunol.,* 1983, vol. 4, 72
- **COLE et al.** Monoclonal Antibodies and Cancer Therapy. Alan R. Liss, Inc, 1985, 77-96
- Current Protocols in Immunology. John Wiley & Sons, 1994
- **GOLD et al.** *Nature,* 1990, vol. 346, 818-822
- **TUERK ; GOLD.** *Science,* 1990, vol. 249, 505-510
- **GOLD et al.** *Annu. Rev. Biochem.,* 1995, vol. 64, 763-797
- **WU ; WU.** *J. Biol. Chem.,* 1988, vol. 263, 14621-4
- **BRIGHMAN et al.** *Am. J. Med. Sci.,* 1989, vol. 298, 278-81
- Ullmann's Encyclopedia of Industrial Chemistry. Marcel Dekker, 1989
- **ANSEL et al.** Pharmaceutical Dosage Forms and Drug Delivery Systems. WILLIAMS & WILKINS, 1994
- American Thoracic Society was adopted by the ATS Board of Directors. *Am. Rev. Respir. Dis.,* November 1986, vol. 136, 225-44
- British guideline on the management of asthma. *Thorax,* 2003, vol. 58 (1), il-94
- **IRIZARRY et al.** *Biostatistics,* 2003, vol. 4, 249-64
- **BOLSTAD et al.** *Bioinformatics,* 2003, vol. 19, 185-93
- **ABECASIS et al.** *Nat. Genet.,* 2002, vol. 30, 97-101
- **SHAM et al.** *Am. J. Hum. Genet.,* 2002, vol. 71, 238-53
- **GUNDERSON et al.** *Nat. Genet.,* 2005, vol. 37, 549-54
- **STEEMERS et al.** *Nat. Methods.,* 2006, vol. 3, 31-3
- **WILLIAMS.** *Biometrics,* 2000, vol. 56, 645-6
- **CLAYTON.** *Am. J. Hum. Genet.,* 1999, vol. 65, 1170-7
- **STOREY ; TIBSHIRANI.** *Proc. Natl. Acad. Sci. USA,* 2003, vol. 100, 9440-5
- **DEVLIN et al.** *Theor. Popul. Biol.,* 2001, vol. 60, 155-66
- **BENJAMINI ; HOCHBERG, J. R.** *Statist. Soc. B.,* 1995, vol. 57, 289-300
- **ASHBURNER et al.** *Nat. Genet.,* 2000, vol. 25, 25-9
- **HARRIS et al.** *Nucleic Acids Res.,* 2004, vol. 32, D258-61
- **MONKS et al.** *Am. J. Hum. Genet.,* 2004, vol. 75, 1094-105
- **ZHU et al.** *Science,* 2004, vol. 304, 1678-82
- **BIERBAUM et al.** *Am. J. Respir. Crit. Care. Med.,* 2005, vol. 172, 1505-9
- **DE BIE et al.** *Clin Exp Allergy,* 2002, vol. 32, 1651-8
- **BURKART et al.** *J. Allergy. Clin. Immunol.,* 2006, vol. 117, 86-91
- **WEILAND et al.** *Eur. Respir. J.,* 2004, vol. 24, 406-12
- **OBER, C. ; HOFFJAN, S.** Asthma genetics 2006: the long and winding road to gene discovery. *Genes Immun,* 2006, vol. 7, 95-100

- **COOKSON, W. ; MOFFATT, M.** Making sense of asthma genes. *N Engl J Med,* 2004, vol. 351, 1794-6
- **SCHADT, E. E. et al.** Genetics of gene expression surveyed in maize, mouse and man. *Nature,* 2003, vol. 422, 297-302
- **MORLEY, M. et al.** Genetic analysis of genome-wide variation in human gene expression. *Nature,* 2004, vol. 430, 743-7
- **CHEUNG, V. G. et al.** Natural variation in human gene expression assessed in lymphoblastoid cells. *Nat Genet,* 2003, vol. 33, 422-5
- **ABECASIS, G. ; CARDON., L. ; COOKSON, W.** Selection strategies for disequilibrium mapping of quantitative traits in nuclear families. *Am J Hum Genet,* 1999, vol. 65, A245
- **DEVLIN, B. ; ROEDER, K. ; WASSERMAN, L.** Genomic control, a new approach to genetic-based association studies. *Theor Popul Biol,* 2001, vol. 60, 155-66
- **MONKS, S. A. et al.** Genetic inheritance of gene expression in human cell lines. *Am J Hum Genet,* 2004, vol. 75, 1094-105
- **ZHU, Z. et al.** Acidic mammalian chitinase in asthmatic Th2 inflammation and IL-13 pathway activation. *Science,* 2004, vol. 304, 1678-82
- **BIERBAUM, S. et al.** Polymorphisms and haplotypes of acid mammalian chitinase are associated with bronchial asthma. *Am J Respir Crit Care Med,* 2005, vol. 172, 1505-9
- **DE BIE, J. J. et al.** Exogenous interleukin-16 inhibits antigen-induced airway hyper-reactivity, eosinophilia and Th2-type cytokine production in mice. *Clin Exp Allergy,* 2002, vol. 32, 1651-8
- **BURKART, K. M. et al.** Association of asthma with a functional promoter polymorphism in the IL16 gene. *J Allergy Clin Immunol,* 2006, vol. 117, 86-91
- **SETAKIS, E. ; STIRNADEL, H. ; BALDING, D. J.** Logistic regression protects against population structure in genetic association studies. *Genome Res,* 2006, vol. 16, 290-6
- **HJELMQVIST, L. et al.** ORMDL proteins are a conserved new family of endoplasmic reticulum membrane proteins. *Genome Biol,* 2002, vol. 3
- American Thoracic Society was adopted by the ATS Board of Directors. *Am Rev Respir Dis,* November 1986, vol. 136, 225-44
- British guideline on the management of asthma. *Thorax,* 2003, vol. 58 (1), i1-94
- **WEILAND, S. K. et al.** Phase II of the International Study of Asthma and Allergies in Childhood (ISAAC II): rationale and methods. *Eur Respir J,* 2004, vol. 24, 406-12
- **GUNDERSON, K. L. ; STEEMERS, F. J. ; LEE, G. ; MENDOZA, L. G. ; CHEE, M. S.** A genome-wide scalable SNP genotyping assay using microarray technology. *Nat Genet,* 2005, vol. 37, 549-54
- **STEEMERS, F. J. et al.** Whole-genome genotyping with the single-base extension assay. *Nat Methods,* 2006, vol. 3, 31-3
- **IRIZARRY, R. A. et al.** Exploration, normalization, and summaries of high density oligonucleotide array probe level data. *Biostatistics,* 2003, vol. 4, 249-64
- **BOLSTAD, B. M. ; IRIZARRY, R. A. ; ASTRAND, M. ; SPEED, T. P.** A comparison of normalization methods for high density oligonucleotide array data based on variance and bias. *Bioinformatics,* 2003, vol. 19, 185-93
- **ABECASIS, G. R. ; CHERNY, S. S. ; COOKSON, W. O. ; CARDON, L. R.** Merlin--rapid analysis of dense genetic maps using sparse gene flow trees. *Nat Genet,* 2002, vol. 30, 97-101
- **SHAM, P. C. ; PURCELL, S. ; CHERNY, S. S. ; ABECASIS, G. R.** Powerful regression-based quantitative-trait linkage analysis of general pedigrees. *Am J Hum Genet,* 2002, vol. 71, 238-53
- **BENJAMINI, Y. ; HOCHBERG, Y.** Controlling the false discovery rate: a practical and powerful approach to multiple testing. *J. R. Statist. Soc. B.,* 1995, vol. 57, 289-300
- **WILLIAMS, R. L.** A note on robust variance estimation for cluster-correlated data. *Biometrics,* 2000, vol. 56, 645-6
- **CLAYTON, D.** A generalization of the transmission/disequilibrium test for uncertain-haplotype transmission. *Am J Hum Genet,* 1999, vol. 65, 1170-7
- **STOREY, J. D. ; TIBSHIRANI, R.** Statistical significance for genomewide studies. *Proc Natl Acad Sci U S A,* 2003, vol. 100, 9440-5
- **ASHBURNER, M. et al.** Gene ontology: tool for the unification of biology. The Gene Ontology Consortium. *Nat Genet,* 2000, vol. 25, 25-9
- **HARRIS, M. A. et al.** The Gene Ontology (GO) database and informatics resource. *Nucleic Acids Res,* 2004, vol. 32, D258-61
- **ABECASIS, G. R. ; COOKSON, W. O.** GOLD--graphical overview of linkage disequilibrium. *Bioinformatics,* 2000, vol. 16, 182-3

SEQUENCE LISTING

<110> COMMISSARIAT A L'ENERGIE ATOMIQUE (CEA)

<120> Method of testing a subject thought to have or be
      predisposed to having asthma, allergie, atopic
      disease or atopic sensitization

<130> 66865/D25269

<160> 70

<170> PatentIn version 3.3

<210> 1
<211> 2109
<212> DNA
<213> Homo sapiens

<400> 1

```
gattcggccg gagctgccag cggggaggct gcagccgcgg gttgttacag ctgctggagc      6
agcagcggcc cccgctcccg ggaaccgttc ccgggccgtt gatcttcggc cccacacgaa     12
cagcagagag gggcagcagg atgaatgtgg gcacagcgca cagcgaggtg aaccccaaca     18
cgcgggtgat gaacagccgt ggcatctggc tctcctacgt gctggccatc ggtctcctcc     24
acatcgtgct gctgagcatc ccgtttgtga gtgtccctgt cgtctggacc ctcaccaacc     30
tcattcacaa catgggcatg tatatcttcc tgcacacggt gaaggggaca ccctttgaga     36
ccccggacca gggcaaggcg aggctgctaa cccactggga gcagatggat tatggggtcc     42
agttcacggc ctctcggaag ttcttgacca tcacacccat cgtgctgtac ttcctcacca     48
gcttctacac taagtacgac cagatccatt ttgtgctcaa caccgtgtcc ctgatgagcg     54
tgcttatccc caagctgccc cagctccacg gagtccggat ttttggaatc aataagtact     60
gagagtgcag ccccttcccc tgcccagggt ggcaggggag gggtagggta aaaggcatgt     66
gctgcaacac tgaagacaga aagaagaagc ctctggacac tgccagagat gggggttgag     72
cctctggcct aatttccccc ctcgcttccc ccagtagcca acttggagta gcttgtagtg     78
gggttggggt aggccccctg ggctctgacc ttttctgaat tttttgatct cttccttttg     84
ctttttgaat agagactcca tggagttggt catggaatgg gctgggctcc tgggctgaac     90
atggaccacg cagttgcgac aggaggccag gggaaaaacc cctgctcact tgtttgccct     96
caggcagcca aagcacttta acccctgcat agggagcaga gggcggtacg gcttctggat    102
tgtttcactg tgattcctag gttttttcga tgccatgcag tgtgtgcttt tgtgtatgga    108
agcaagtgtg ggatgggtct ttgcctttct gggtagggag ctgtctaatc caagtcccag    114
gcttttggca gcttctctgc aacccaccgt gggtcctggt tgggagtggg gagggtcagg    120
ttggggaaag atggggtaga gtgtagatgg cttggttcca gaggtgaggg ggccaggct    126
gctgccatcc tggcctggtg gaggttgggg agctgtagga gagctagtga gtcgagactt    132
agaagaatgg ggccacatag cagcagagga ctggtgtaag ggagggaggg gtagggacag    138
aagctagacc caatctcctt tgggatgtgg gcagggaggg aagcaggctt ggagggttaa    144
tttacccaca gaatgtgata gtaatagggg agggaggctg ctgtgggttt aactcctggg    150
ttggctgttg ggtagacagg tggggaaaag gcccgtgagt cattgtaagc acaggtccaa    156
cttggccctg actcctgcgg gggtatgggg aagctgtgac agaaacgatg ggtgctgtgg    162
tcctctgcag gccctcaccc cttaacttcc tcatgcagac tggcactggg cagggcctct    168
catgtggcag ccacatgtgg cgttgtgagg ccaccccatg tggggtctgt ggtgagagtc    174
ctgtaggatc cctgctcaag cagcacagag gaaggggcaa gacgtggcct gtaggcactg    180
tctcagcctg cagagaagaa agtgaggccg ggagcctgag cctgggctgg agccttctcc    186
cctccccagt tggactaggg gcagtgttaa ttttgaaaag gtgtgggtcc ctgtgtcctt    192
ttccaggggt ccaagggaac aggagaggtc actggcctg ttttctccct cctgaccctg    198
catctcccac cctgtgtatc atagggaact ttcaccttaa aatctttcta agcaaagtgt    204
gaataggatt tttactccct ttgtacagta ttctgaggaa cgcaaataaa agggcaacat    210
gtttctgtt                                                            2109
```

<210> 2
<211> 153

```
<212> PRT
<213> Homo sapiens

<400> 2

Met Asn Val Gly Thr Ala His Ser Glu Val Asn Pro Asn Thr Arg Val
1               5                   10                  15
Met Asn Ser Arg Gly Ile Trp Leu Ser Tyr Val Leu Ala Ile Gly Leu
            20                  25                  30
Leu His Ile Val Leu Leu Ser Ile Pro Phe Val Ser Val Pro Val Val
            35                  40                  45
Trp Thr Leu Thr Asn Leu Ile His Asn Met Gly Met Tyr Ile Phe Leu
        50                  55                  60
His Thr Val Lys Gly Thr Pro Phe Glu Thr Pro Asp Gln Gly Lys Ala
65                  70                  75                  80
Arg Leu Leu Thr His Trp Glu Gln Met Asp Tyr Gly Val Gln Phe Thr
                85                  90                  95
Ala Ser Arg Lys Phe Leu Thr Ile Thr Pro Ile Val Leu Tyr Phe Leu
            100                 105                 110
Thr Ser Phe Tyr Thr Lys Tyr Asp Gln Ile His Phe Val Leu Asn Thr
            115                 120                 125
Val Ser Leu Met Ser Val Leu Ile Pro Lys Leu Pro Gln Leu His Gly
        130                 135                 140
Val Arg Ile Phe Gly Ile Asn Lys Tyr
145                 150
```

```
<210> 3
<211> 255
<212> DNA
<213> Homo sapiens


<220>
<221> misc_feature
<222> (128)..(128)
<223> n = c or t

<220>
<221> misc_feature
<222> (160)..(160)
<223> n = a, t, g or c

<400> 3
caggtactgt tctaggtact gtaaatatct agcaaggaaa aaaatagccc aagaaggctg       6
gaaggagagt agacactaaa caaaactcca aagttatctg tgttcagtgt catgcgatga      12
attaaaanag ggctgtgtta aaatagtgac tgaattgtcn ggaaaagctt ttctgaagaa      18
atgacattta agatgagatc tgagtaacag agtaacattc ataaagcaaa agtgattaga      24
tttatcaaca taaat                                                      255
```

```
<210> 4
<211> 255
<212> DNA
<213> Homo sapiens


<220>
<221> misc_feature
<222> (128)..(128)
<223> n = g or t
```

```
<400> 4
tctttttttt ttaaagccta tcgggaacct gattattcat atcagatggc tgtacgtttt       6
accacaaggt cttgtatagg gagatacaat gatgtattct ttagagtgct gaagaaaatc      12
ttggatantc taatttctga tttgtcatgc catgtcatag agccatcata taaatgccat      18
tctgttgaaa ttccagaaca tggcctcata catgagctat ttatagcatt tcaaggtaaa      24
attttaaaa tttct                                                         255


<210> 5
<211> 255
<212> DNA
<213> Homo sapiens


<220>
<221> misc_feature
<222> (128)..(128)
<223> n= a or g


<400> 5
tccctcagaa agaagagcac agataaactg acagattagt gtgattacct ttagctctgt       6
gagtggaaag cttgacagtc aaaacaaaaa cctgatctca agaaaaaata tttgtaacgt      12
tataaatngg gaaaaacgtt tatatcactg ccaggtttgt gcagatgtca gtggcctaga      18
gtggaaattc tgggtctcac tgtagcaagc tgcttatttc accagtcatc cgttactcag      24
aattgaacat taatt                                                        255


<210> 6
<211> 255
<212> DNA
<213> Homo sapiens


<220>
<221> misc_feature
<222> (128)..(128)
<223> n = t or c

<220>
<221> misc_feature
<222> (155)..(155)
<223> n = a, t, g or c

<220>
<221> misc_feature
<222> (238)..(238)
<223> n = a, t, g or c

<400> 6
ccagatacag gttttctctc gtattaattg atcggaatga aaagggtagt tccctcttca       6
gcttcctgga gagagtggag agagatgaga gttaaggatc tcagggcctt acctcccact      12
gactcttntc cctctctgtg agccagctcc tattnaagat taatcgcttc tgagactgga      18
aaaccaagga acacagcacc ccatcgcagt tcactttacc cacctcgggg tggaggtngg      24
ggtggggtac aaagt                                                        255


<210> 7
<211> 255
<212> DNA
<213> Homo sapiens
```

```
<220>
<221> misc_feature
<222> (128)..(128)
<223> n = c or t


<220>
<221> misc_feature
<222> (250)..(250)
<223> n = a, t, g or c


<400> 7
agatcaagcc actgcactcc agcctgggcg acagcgagac tccatctcca ataaaagcag      6
ttctgtcgct gttgtttgta tgaaccacac aagtatgaag tgaggcaacc ctggaaagtc     12
acaaacangc atggactcgg ccctgattga tcaggcacta ataagggcct tggttctgag     18
tcagttgcca ccccaggttc gtggaggatg tgtgggcatg ccgaaggggc tgcgtgcctt     24
tgtctccaan ctccc                                                     255


<210> 8
<211> 255
<212> DNA
<213> Homo sapiens


<220>
<221> misc_feature
<222> (128)..(128)
<223> n = t or c


<400> 8
atagagcagc actggtaaca tcctctggca ccagactgcc agggttcaac tcgcagctcc      6
tctagttacc agctgtgtaa ccttaagcaa gttactgaac ctctctgtgc ttcagttacc     12
tcgttgcnaa aagaattggc aagtgacact ggtgttcgga aaaaataaag taaatgaaga     18
tggcgatgat catgataata ccctcctcat gttgtggata ttaaatgggt cagtaaatgt     24
gaaatgaaac actca                                                     255


<210> 9
<211> 255
<212> DNA
<213> Homo sapiens


<220>
<221> misc_feature
<222> (128)..(128)
<223> n = t or c


<400> 9
cacaggccaa gacagagcgt gagactcccc ggtgatggct tttccagacc catgccatgt      6
gtccctcagg tctttatcat ctcacaccta gacctttgcc ataactggtt ttcctgccac     12
caatacancc ccgacacaac caacagagtg atccagacat caaatctaat tttaaaacct     18
ttcaatccga ccagatgcgg tggctcacgc ttgtaatccc aacactttgg gaagtcgagg     24
agggtggatc acttg                                                     255


<210> 10
<211> 255
<212> DNA
```

<213> Homo sapiens


<220>
<221> misc_feature
<222> (128)..(128)
<223> n = g or a

<400> 10

```
aaggggaggg gaggcggcct cccaagggac ggttcctact ctgagccaag ccctcagagc        6
tgagctgaag tctggaagtt tggagtcttc catttctgtt tgggctctgc cccccaaacc       12
agctggcnac tccaccccct cctgggtccc tcccaatccc tattccacat ccctgctgt       18
gtggctccct aatgcctgca cctacgtgtc ccctcctgga ggcaacacta tatatattgg       24
gtgcaggggc gggtg                                                      255
```


<210> 11
<211> 255
<212> DNA
<213> Homo sapiens


<220>
<221> misc_feature
<222> (128)..(128)
<223> n = g or a

<220>
<221> misc_feature
<222> (252)..(252)
<223> n = a, t, g or c

<400> 11

```
gcctatatcc cgggctcctt ggcagccttg gcagccacct tgacactctc ctgtctcccc        6
acctccacag agacaatgac catgtttgaa aatgtcaccc gggccctggc cagacagcta       12
aaccctcnag gggacctgac accacttgac agcctcatcg acttcaagcg cttccatccc       18
ttctgcctgg tgctgaggaa gaggaagagc acgctcttct gggggggcccg gtacgtccgc       24
accgactaca cnctg                                                      255
```


<210> 12
<211> 255
<212> DNA
<213> Homo sapiens


<220>
<221> misc_feature
<222> (64)..(66)
<223> n = a, t, g or c

<220>
<221> misc_feature
<222> (128)..(128)
<223> n = c or t

<220>
<221> misc_feature
<222> (194)..(194)
<223> n = a, t, g or c

```
<220>
<221> misc_feature
<222> (245)..(245)
<223> n = a, t, g or c

<400> 12
taatcccccca aggagcctct gcttttgttt cccaagtccc taagaatacc tagcctcaac     6
tttnnnaaga cagatagagg catataaata ctgtcccaga gagatataga aactgacaga    12
cagagacngg aaggcacagt cagaagcaga agtaagactt ggagactgag tgtgttcccg    18
aatcatgggg gtgnatgagc tagcataatc tccctgtgtt tttataatga tcactgatga    24
aactnccttg aagac                                                    255


<210> 13
<211> 255
<212> DNA
<213> Homo sapiens


<220>
<221> misc_feature
<222> (128)..(128)
<223> n = a or g

<400> 13
acataaagtt cactgattag ggttagctgt ggcctatgca gtactaaagg tcaaaaaaaa     6
aagatctatt tgaaaaatta ccgaacctttt ctcctctcct gttttcatta ctccttatgt   12
gggacaanct gcctttaact taagacacat ttatctgcta atgatagaat aaggaagatt    18
aacattaaaa ctaacattaa aaaaaactca acattaacaa caccattaat cacaacagtc    24
aaaaggttaa aacaa                                                    255



<210> 14
<211> 255
<212> DNA
<213> Homo sapiens



<220>
<221> misc_feature
<222> (128)..(128)
<223> n = c or t

<400> 14
tctggagggc actgttgtgt ggggaccctg tgaggtttgc aggaggcggg caaaggggca     6
ccagacaggg aacatgcaca caggcagcct ggccatggag agctcctggc tgtccgccca    12
ggtggcgnct tgggcagagc tggcgctcca caagtgatta ttgatggtga caggattccc    18
tcatcctgct ccaacctctc aactgtggac acattcaccg gcagtcactc atctcacatg    24
cccaccagca cgcgc                                                    255


<210> 15
<211> 255
<212> DNA
<213> Homo sapiens


<220>
<221> misc_feature
<222> (128)..(128)
<223> n = a or g
```

```
<220>
<221> misc_feature
<222> (170)..(170)
<223> n = a, t, g or c

<400> 15
gaggagggtg gggggtggta acagaagggg accccctgta tccctaaggc ctgtggtccc      6
tggtctccca ggtagacaga gggcttcagg acccagccag gcagctgggg atgtgggggag     12
gaggctgntg gggaggaaac aggcagctgt cctcccggta gagctaagan cacactcagt      18
tccaccccag ttcagctcca ggaggtagag gggctgccca ctctcgagtc ccagatgctg      24
ggaggccttc cctag                                                        255


<210> 16
<211> 255
<212> DNA
<213> Homo sapiens


<220>
<221> misc_feature
<222> (86)..(86)
<223> n = a, t, g or c

<220>
<221> misc_feature
<222> (128)..(128)
<223> n = a or g

<400> 16
cctaaggcct gtggtccctg gtctcccagg tagacagagg gcttcaggac ccagccaggc      6
agctggggat gtggggagga ggctgntggg gaggaaacag gcagctgtcc tcccggtaga     12
gctaaganca cactcagttc caccccagtt cagctccagg aggtagaggg gctgcccact     18
ctcgagtccc agatgctggg aggccttccc tagaacagac tccaaggctg gtgagggcca     24
acagggggtg ggctg                                                       255


<210> 17
<211> 255
<212> DNA
<213> Homo sapiens


<220>
<221> misc_feature
<222> (128)..(128)
<223> n = t or c

<400> 17
cagaactgga aactccactt tactacatac tggctgtgtg accttgggca gggcatttaa      6
tctctctaat ccccatctgt aaaactggga ttagagaaac tatctcaaag ggtgtgataa     12
aaattacntg acatattgtg acacctgtca cacagtgagt gctcaatacg ttggttcttt     18
gcccactatg gtccaaatcg aatgtaccag tccaaagtcc ctccttcaaa caaaacgttc     24
caagatgatc cctgc                                                       255


<210> 18
<211> 255
<212> DNA
<213> Homo sapiens
```

```
<220>
<221> misc_feature
<222> (128)..(128)
<223> n = t or c


<400> 18
tctgtcttgt ctccctctgc ttataggttg tgcctctggt ttgggggcct ctcagcctgt     6
ctgggtccct cccttgctgt gcagttggcc tcgtggcctc tgctgctgtt tgtgcctctc    12
tctgttanta acccgtcctc tcgctgttag acatctctct cactgcctgt ctctggttct    18
gtcctcaggc cacccctgtt ctccgatgtg taagggctcc cgctgctggg gagagagttc    24
tgaggattgt cagag                                                    255



<210> 19
<211> 255
<212> DNA
<213> Homo sapiens


<220>
<221> misc_feature
<222> (128)..(128)
<223> n = t or c


<400> 19
agggtgaggc ttcccagcaa ggtcagctgg tcagctcttc ctctttgtgg atttgcatgt     6
atgactgaaa ggcttttcca cgtgtggttg atatatctca gaaagaatgt ttcatatagc    12
acatctcngg gaccactcat tccactgctg tagaagataa tgaccaaata cctttttggc    18
ttttaaattc cataaaattc aagtccctga tgggaaaaca aaggtttcac aaaagtaata    24
atatgctaga cctgc                                                    255



<210> 20
<211> 255
<212> DNA
<213> Homo sapiens


<220>
<221> misc_feature
<222> (128)..(128)
<223> n = g or a


<220>
<221> misc_feature
<222> (219)..(219)
<223> n = a, t, g or c


<220>
<221> misc_feature
<222> (221)..(221)
<223> n = a, t, g or c


<400> 20
cgaaagggtc acggaagccg tggcagccca tgtgaatcgt gaacatcaca tagtccagga     6
agaggacgcg gcagtggtca caccgataca catccatcac ctcccccttcc ttgttgatca    12
ctttgacnga gtctcttggg cagatgggcg ggggcttgag gagttcgtaa gagcggggaa    18
cctccttcag aagtggcatc ccattgcggg cccgagacng naccatgtga ttttgctgat    24
agatgtgatt ctggc                                                    255
```

```
<210> 21
<211> 255
<212> DNA
<213> Homo sapiens


<220>
<221> misc_feature
<222> (128)..(128)
<223> n = c or t

<220>
<221> misc_feature
<222> (175)..(175)
<223> n = a, t, g or c

<400> 21
aggacacgta atcaacatcc tctacatttg ggaggatgct gaagtgtgga cacattgacc      6
taaataactg taattagggc aggtcactaa atggcaaact tttgagataa catcaccttt     12
tattcctngt tacccaccaa actggtcttt agagtcattt ccagataaac attantctgt     18
aaatcagtga ccaaatgcct gacatttaaa aacataaaat tctgcagtgt tcatggatgg     24
acatattttg aggca                                                     255


<210> 22
<211> 255
<212> DNA
<213> Homo sapiens


<220>
<221> misc_feature
<222> (88)..(88)
<223> n = a, t, g or c

<220>
<221> misc_feature
<222> (128)..(128)
<223> n = c or t

<220>
<221> misc_feature
<222> (216)..(216)
<223> n = a, t, g or c

<400> 22
gcattttgtg gaccacagtt tgcaagtagt acgtctggat agctgtcgac caggctttgg      6
aaaaaatgaa cgtctacaca gtaattgngc tagctgttgt ggtaactata aaatataaat     12
atctaaanat ttaggttata gagtcagaag aatatataag taattcactt ggcataactg     18
aatagaatct ttacagtgat tctcttacca aaaaanatat atatatttag ctaaaaggaa     24
gtaaaaattc aaagc                                                     255


<210> 23
<211> 255
<212> DNA
<213> Homo sapiens
```

<220>
<221> misc_feature
<222> (128)..(128)
<223> n = a or c

<400> 23
aaaatcagta agaccaaacc actggaaaac atgcattttg gaaactttaa aataaatggt      6
taacatggca tttctaagaa ggcatttaat ccagtatctc tagtgtacaa aggaaacttg     12
aagttttnat ggattatttt taatgaaatg ttttattgtt tacaaacggt atgttgctgt     18
gtacctaaga gtatagtaag gtcaagaaga gtatcaaagt ataataaaat aaaaattgta     24
tactctccat atatt                                                     255


<210> 24
<211> 255
<212> DNA
<213> Homo sapiens


<220>
<221> misc_feature
<222> (128)..(128)
<223> n = a or g

<400> 24
tccctcagaa agaagagcac agataaactg acagattagt gtgattacct ttagctctgt      6
gagtggaaag cttgacagtc aaaacaaaaa cctgatctca agaaaaaata tttgtaacgt     12
tataaatngg gaaaaacgtt tatatcactg ccaggtttgt gcagatgtca gtggcctaga     18
gtggaaattc tgggtctcac tgtagcaagc tgcttatttc accagtcatc cgttactcag     24
aattgaacat taatt                                                     255


<210> 25
<211> 255
<212> DNA
<213> Homo sapiens


<220>
<221> misc_feature
<222> (128)..(128)
<223> n = t or c

<400> 25
tttacactgc cacaacacac aaaaaaactt caaatagaac agtaagcatt accattctct      6
tgttttttca ccttttatat tgtgaagtac atcatggata cagaaaagtg tatttaatat     12
aaaaccgnag cttaatgaat aattaccaaa taaactcctg agctacccgc cacccaggtg     18
aagaaataaa atattgttag cattcttgaa acccacccct ttcagatgct caaccccttt     24
cagatatgat tctcc                                                     255


<210> 26
<211> 255
<212> DNA
<213> Homo sapiens


<220>
<221> misc_feature
<222> (120)..(120)
<223> n = a, t, g or c

```
<220>
<221> misc_feature
<222> (128)..(128)
<223> n = g or a

<400> 26
tgaaatgatc ccatcatttt tcttcttaaa ttgctagtgt ggtaaattac attaattttt      6
caatgttaag ccagccttga aatcccagat aaatccagtt tatattatta tttttctacn     12
gtgatttngt ttgctgattt gttttctgt tttttcatct aagctcacga atgagactgg      18
cctgcatttt cctttctctt attgttcttg ttaagttttg ctagtgttgt ggatgaattg     24
gaaagtgtct ctgaa                                                     255


<210> 27
<211> 255
<212> DNA
<213> Homo sapiens


<220>
<221> misc_feature
<222> (36)..(36)
<223> n = a, t, g or c

<220>
<221> misc_feature
<222> (71)..(71)
<223> n = a, t, g or c

<220>
<221> misc_feature
<222> (128)..(128)
<223> n = g or a

<220>
<221> misc_feature
<222> (149)..(149)
<223> n = a, t, g or c

<400> 27
caccccagcc tacagcgaga cccttcctca cctagnaggg catccaggaa gtccagaatg      6
gcttttgcac ncgcttctac caagacccca gcagcattaa aaaggctgct taggagaggc     12
ttgtctgngt cctccatgtg tagctcccng gaaatcagga cctcagatac ctagggccag     18
gaagtgtggg agatgagcag cagtctcacc atagcagatt atcctcactg tgccaactgc     24
catcccctct ggctc                                                     255


<210> 28
<211> 255
<212> DNA
<213> Homo sapiens


<220>
<221> misc_feature
<222> (15)..(15)
<223> n = a, t, g or c

<220>
<221> misc_feature
```

```
<222> (50)..(50)
<223> n = a, t, g or c

<220>
<221> misc_feature
<222> (107)..(107)
<223> n = a, t, g or c

<220>
<221> misc_feature
<222> (128)..(128)
<223> n = c or t

<220>
<221> misc_feature
<222> (237)..(237)
<223> n = a, t, g or c

<400> 28
ccttcctcac ctagnagggc atccaggaag tccagaatgg cttttgcacn cgcttctacc      6
aagaccccag cagcattaaa aaggctgctt aggagaggct tgtctgngtc ctccatgtgt     12
agctcccngg aaatcaggac ctcagatacc tagggccagg aagtgtggga gatgagcagc     18
agtctcacca tagcagatta tcctcactgt gccaactgcc atcccctctg gctcctntct     24
ctctctgccc caagg                                                     255


<210> 29
<211> 255
<212> DNA
<213> Homo sapiens


<220>
<221> misc_feature
<222> (94)..(94)
<223> n = a, t, g or c

<220>
<221> misc_feature
<222> (128)..(128)
<223> n = g or a

<220>
<221> misc_feature
<222> (139)..(139)
<223> n = a, t, g or c

<400> 29
aattaggatt tggagaaagt taaagcagtg gttctcaacc ttgactacat gagaatcacc      6
tgaggagtta ttacaaatcc tgatgtccag gccncacccc cacaattaag tcagagtttc     12
tggagacngg actcagtcnt cactagtttt aaagtttcct ggtgtgattt tggggtataa     18
ccaatgagaa ccactgtgtt tagaggagac agatgcagcc tccagactaa agttgaaacc     24
tgctaggcag tgcct                                                     255


<210> 30
<211> 255
<212> DNA
<213> Homo sapiens
```

```
<220>
<221> misc_feature
<222> (101)..(101)
<223> n = a, t, g or c

<220>
<221> misc_feature
<222> (128)..(128)
<223> n = g or a

<220>
<221> misc_feature
<222> (211)..(211)
<223> n = a, t, g or c

<400> 30
ttgatcggaa tgaaaagggt agttccctct tcagcttcct ggagagagtg gagagagatg      6
agagttaagg atctcagggc cttacctccc actgactctt ntccctctct gtgagccagc     12
tcctattnaa gattaatcgc ttctgagact ggaaaaccaa ggaacacagc accccatcgc     18
agttcacttt acccacctcg gggtggaggt nggggtgggg tacaaagtag ggtgagaggc     24
caggtacggt ggcta                                                     255


<210> 31
<211> 255
<212> DNA
<213> Homo sapiens


<220>
<221> misc_feature
<222> (128)..(128)
<223> n = c or t

<400> 31
agctccttgc gctggtccct ggagccagct gcatcatctg cccttaggct catgaagcat      6
cagattccat tcccgctggg ggaaactcat gttgagacct aaaacagtct gatggagttt     12
ctatttgnaa tcaaaaggcc ctcaagacat gggtccctgc ctccgatgac tttcccctac     18
ctatccagtg cccccacccca catacagata ccaataccct cacctgacaa acaagcattc    24
tataatagaa aacac                                                     255


<210> 32
<211> 255
<212> DNA
<213> Homo sapiens


<220>
<221> misc_feature
<222> (128)..(128)
<223> n = c or t

<220>
<221> misc_feature
<222> (205)..(250)
<223> n = a, t, g or c

<400> 32
agatcaagcc actgcactcc agcctgggcg acagcgagac tccatctcca ataaaagcag      6
ttctgtcgct gttgtttgta tgaaccacac aagtatgaag tgaggcaacc ctggaaagtc     12
```

33

```
acaaacangc atggactcgg ccctgattga tcaggcacta ataagggcct tggttctgag      18
tcagttgcca ccccaggttc gtggaggatg tgtgggcatg ccgaaggggc tgcgtgcctt      24
tgtctccaan ctccc                                                      255
```

```
<210> 33
<211> 255
<212> DNA
<213> Homo sapiens
```

```
<220>
<221> misc_feature
<222> (50)..(50)
<223> n = a, t, g or c
```

```
<220>
<221> misc_feature
<222> (113)..(113)
<223> n = a, t, g or c
```

```
<220>
<221> misc_feature
<222> (128)..(128)
<223> n = g or a
```

```
<400> 33
agaatgtact ttgtaagcct atcccaaaca tataagaact aatgataatn ccaccaccct       6
ttgctgaccc ctttttttgga ctcagcttgc ctgcacccag ccttgttgct canataaagc     12
ccgtttcntg gactctcttc acatggacgc acgtgacatg aggtcaagga agagccaaag      18
gttccaggac aatcagtacc tggaatgatt tgcctgcctt acttctagaa cgtgaggcag      24
ccatgactca gtaaa                                                      255
```

```
<210> 34
<211> 255
<212> DNA
<213> Homo sapiens
```

```
<220>
<221> misc_feature
<222> (128)..(128)
<223> n = c or t
```

```
<400> 34
accagggagg aatgacgggc acggttgcca gacttcacat tactaacctc aactggtcgt       6
ttcttccctt ggctctgatt cttgggctcc ctgccttgct gtattctgca aggcaattgg      12
atgggtgnct ccctaccaac aatttcccaa tgaggtggtg gtaaattggc ctcagaagaa      18
acaaaagctt cctgggatcc acttccccat cctacctttg gccaccttcc cctatatccc      24
ttctgcccag cacag                                                      255
```

```
<210> 35
<211> 255
<212> DNA
<213> Homo sapiens
```

```
<220>
<221> misc_feature
```

```
<222> (48)..(48)
<223> n = a, t, g or c

<220>
<221> misc_feature
<222> (93)..(93)
<223> n = a, t, g or c

<220>
<221> misc_feature
<222> (128)..(128)
<223> n = g or a

<220>
<221> misc_feature
<222> (139)..(139)
<223> n = a, t, g or c

<220>
<221> misc_feature
<222> (180)..(180)
<223> n = a, t, g or c

<220>
<221> misc_feature
<222> (201)..(201)
<223> n = a, t, g or c

<400> 35
agacacaggg aaacagaaac atgttgccct tttatttgcg tttctcanaa tactgtacaa          6
agggagtaaa aatcctattc acactttgct tanaaagatt ttaaggtgaa agttccctat         12
gatacacngg gtgggagang cagggtcagg agggagaaaa caggcccagt gacctctccn         18
gttcccttgg acccctggaa naggacacag ggacccacac cttttcaaaa ttaacactgc         24
ccctagtcca actgg                                                          255


<210> 36
<211> 255
<212> DNA
<213> Homo sapiens

<220>
<221> misc_feature
<222> (128)..(128)
<223> n = g or a

<220>
<221> misc_feature
<222> (175)..(175)
<223> n = a, t, g or c

<220>
<221> misc_feature
<222> (210)..(210)
<223> n = a, t, g or c

<400> 36
ggtcatcaag aggcacgcag ccccagggca gagaagtcaa ggcccatccc ccagcctcac          6
cccctatggc actcccatga ggaccctctg ccgacaaagc cccagggaga gaattctcac         12
tcagcacnac ccctgctgga ctgtgtctca ctgaaggcct aatgtgggac atctnctctt         18
```

```
aagctcttcc catcaggagg gaaaggaagn gaggcctgtg ggtacaggca gcagtggatg   24
aactattttg aggtc                                                    255


<210> 37
<211> 255
<212> DNA
<213> Homo sapiens


<220>
<221> misc_feature
<222> (81)..(81)
<223> n = a, t, g or c

<220>
<221> misc_feature
<222> (128)..(128)
<223> n = g or a

<220>
<221> misc_feature
<222> (163)..(163)
<223> n = a, t, g or c

<400> 37
cccccagcct caccccctat ggcactccca tgaggaccct ctgccgacaa agccccaggg    6
agagaattct cactcagcac nacccctgct ggactgtgtc tcactgaagg cctaatgtgg   12
gacatctnct cttaagctct tcccatcagg agggaaagga agngaggcct gtgggtacag   18
gcagcagtgg atgaactatt ttgaggtcac tgcatccact cacctcctct gatttttca   24
attctggatc tcccc                                                    255


<210> 38
<211> 255
<212> DNA
<213> Homo sapiens


<220>
<221> misc_feature
<222> (128)..(128)
<223> n = t or c

<400> 38
ctgcaggatt tttccactgc acctggctgc ctccgccctg gccccagatt ggctcctcca    6
gaaacagact agcccggccc tttgccctgc tgggccctcc tgggagagcg cttttcacaa   12
cattggcngg gatgagggtc tggagggccc ggaacggagc ctgccttctc cttctcagtc   18
acgcttggcc caggctgccc cggaccgggc cacatccttg gccgccccgc acctgacgtg   24
agaagaggct acgta                                                    255


<210> 39
<211> 255
<212> DNA
<213> Homo sapiens


<220>
<221> misc_feature
<222> (124)..(124)
```

<223> n = a, t, g or c

<220>
<221> misc_feature
<222> (128)..(128)
<223> n = a or c

<400> 39
tctgcctatg tcctgtggaa gtttctcctg gtttgtggag gtgcctttcc ttatagtcat     6
ctttctgcag acataagcaa acccagtgtt gttcttttca cagaaacctc cccagcatta    12
aggnaagnaa ggtgtctctg ttttttccga agtgtgcctg cttgactcct ttgatttttc    18
tctgggattc ttcatcctcc atcaagttga tcgatccctc tggatcttgg tcagtttcct    24
gacatctcta tgaag                                                    255


<210> 40
<211> 255
<212> DNA
<213> Homo sapiens


<220>
<221> misc_feature
<222> (128)..(128)
<223> n = t or c

<220>
<221> misc_feature
<222> (197)..(197)
<223> n = a, t, g or c

<400> 40
tgcactatta ataactcctg ttatcgagga tttttttttc acaataggaa tgaacagagt     6
tctgactcac tctaggtttg tggtaaattg cagatcactt tctgctttat ttagcgggag    12
tccctgtngt catctttctc cttctctctt ggccctcata attatcatat cttcccccaaa   18
cgttcttcag ctgtgantag ccctaagcac tattcagagt gagtgacagc tctcttattt    24
cgtgctgggg gacat                                                    255


<210> 41
<211> 255
<212> DNA
<213> Homo sapiens


<220>
<221> misc_feature
<222> (59)..(59)
<223> n = a, t, g or c

<220>
<221> misc_feature
<222> (128)..(128)
<223> n = a or c

<400> 41
ctctaggttt gtggtaaatt gcagatcact ttctgcttta tttagcggga gtccctgtng     6
tcatctttct ccttctctct tggccctcat aattatcata tcttccccaa acgttcttca    12
gctgtganta gccctaagca ctattcagag tgagtgacag ctctcttatt tcgtgctggg    18
ggacatacaa acccttggac aaacacttgt cactgtgtgc caagaagctg aaaaattctt    24
cttgcctcag agcca                                                    255

```
<210> 42
<211> 255
<212> DNA
<213> Homo sapiens


<220>
<221> misc_feature
<222> (128)..(128)
<223> n = t or c

<400> 42
atagagcagc actggtaaca tcctctggca ccagactgcc agggttcaac tcgcagctcc      6
tctagttacc agctgtgtaa ccttaagcaa gttactgaac ctctctgtgc ttcagttacc     12
tcgttgcnaa aagaattggc aagtgacact ggtgttcgga aaaaataaag taaatgaaga     18
tggcgatgat catgataata ccctcctcat gttgtggata ttaaatgggt cagtaaatgt     24
gaaatgaaac actca                                                     255


<210> 43
<211> 255
<212> DNA
<213> Homo sapiens


<220>
<221> misc_feature
<222> (128)..(128)
<223> n = a or g

<400> 43
attgcttgaa cccgggaggc ggaggctgca gagagcagag atggcgccat tttactccag      6
cctgggcaac aagagtggaa ctttgtctca aaaaataaag aaaggaagga aggaaggaag     12
aaagacanca acaaaaaaac cttgaatcag gatgaaggga tttagggtga tctttttcat     18
tctctagttt caaacttcac gtaatgtggt attactttta aattttttat taaaattgtt     24
taaaaacaaa atgag                                                     255


<210> 44
<211> 255
<212> DNA
<213> Homo sapiens


<220>
<221> misc_feature
<222> (128)..(128)
<223> n = a or g

<400> 44
aggcctacct cacctgtcac cttctcggtg cagccttccc cagtgtccaa gcagagtcca      6
ctgcttcatc ctccaggctg ctgtgaccct ggcggatccc tcaacccaag ccttcccttg     12
ggtgatcnga gggctatatt tgtgcacaca ccagtcttcc ctcattggct gggggacatt     18
tagttctcag ccaggcgcgg tggctctaca caaaaataca aaagttagct gggtgtggtg     24
gtgtgcgcct gtagt                                                     255


<210> 45
<211> 255
```

```
<212> DNA
<213> Homo sapiens


<220>
<221> misc_feature
<222> (26)..(26)
<223> n = a, t, g or c


<220>
<221> misc_feature
<222> (128)..(128)
<223> n = c or g


<400> 45
tggccaacat ggcgaaacct catctntgct aaaaatacaa aaattacctg ggtgtggtga      6
tgcaggcctg taatcccagc tactcaggag gctgaggcag gagaatcact tgaatctggg     12
aagtgaangt tgtagtgaag cgagatcatg caattgcact ccagcctggg tgcactcaga     18
gagagactcc agactctgca ctccagagag agactccatc tcaaagaaaa aaaaaaaaaa     24
aaaggtaggg agagg                                                    255


<210> 46
<211> 255
<212> DNA
<213> Homo sapiens


<220>
<221> misc_feature
<222> (128)..(128)
<223> n = c or t


<400> 46
aggaggtctg ggcactgcga ggagccagga aatgaggaac cctggacaga catgttttcc      6
aaggaaagac taggggaagt ttgctcaccg tggaagaagg ggccaccgag catggaagaa     12
tgtgccangg cgtgaaagaa tggcagaagg gagtagggag aggaatggag ttgggggaat     18
acaggttgct ggagacactg gatgtgagat ggggcccacc cagttctgcc cgccaggcct     24
gccaagcagg gatcc                                                    255


<210> 47
<211> 255
<212> DNA
<213> Homo sapiens


<220>
<221> misc_feature
<222> (128)..(128)
<223> n = c or t


<220>
<221> misc_feature
<222> (226)..(226)
<223> n = a, t, g or c


<400> 47
tcgggggtgc tcctgctggg ctggaggcca gcttcccatc tccccttctg ctccccagcc      6
cctgccacca gagtccatct gctggactgg ccaggactgt gccgacacac tcagagcggc     12
tcctgcangg aacgactgcc ccagaatccc ggactgctcc ctctgcatcc tgaccccggg     18
```

```
cttgtgcttg tccactgggc aggcaggagg gggtgacgta actggnttca ctcccatcct      24
cacctctgcc atgga                                                      255
```

<210> 48
<211> 255
<212> DNA
<213> Homo sapiens

<220>
<221> misc_feature
<222> (128)..(128)
<223> n = g or a

<400> 48
```
aaggggaggg gaggcggcct cccaagggac ggttcctact ctgagccaag ccctcagagc       6
tgagctgaag tctggaagtt tggagtcttc catttctgtt tgggctctgc cccccaaacc      12
agctggcnac tccacccccct cctgggtccc tcccaatccc tattccacat ccctgctgt      18
gtggctccct aatgcctgca cctacgtgtc ccctcctgga ggcaacacta tatatattgg      24
gtgcaggggc gggtg                                                      255
```

<210> 49
<211> 255
<212> DNA
<213> Homo sapiens

<220>
<221> misc_feature
<222> (81)..(81)
<223> n = a, t, g or c

<220>
<221> misc_feature
<222> (128)..(128)
<223> n = g or a

<400> 49
```
ccttggcgca ggacacctct tccgtttggg ctttccgtcc tcgtgatttc gctctccatc       6
tgacagtcat ccatggtgac ngggtgtgtc cctttcccct aaacaggatc ctcaatgccc      12
atcggaanac gttgtaaggc gttgagcccc caacgccccc tcaccacaag accctgctgt      18
gaccatcttt tcctggtgag gagagtctgc acggggagc agggcgccat accccagaaa       24
gaaacctctg tgcct                                                      255
```

<210> 50
<211> 255
<212> DNA
<213> Homo sapiens

<220>
<221> misc_feature
<222> (128)..(128)
<223> n = g or c

<220>
<221> misc_feature
<222> (248)..(248)

<223> n = a, t, g or c

<400> 50

```
ctcctgacct caagtgatcc gctcccctca gcttcccaaa gtgctgggat tacaggtgtg      6
aaccaccaca cccggccccc agagccactt tacaatgccc tgcccacaga aggagggaga     12
gcatgccntg tccctcatgc cctttcctct cctttaccct ctgcagagct gagtgaggtg     18
caacagcagc tgctggccat gtccagggag aagggatcc tttcccaata ggtggagctg      24
gagagggncc tgggg                                                      255
```

<210> 51
<211> 255
<212> DNA
<213> Homo sapiens

<220>
<221> misc_feature
<222> (8)..(8)
<223> n = a, t, g or c

<220>
<221> misc_feature
<222> (128)..(128)
<223> n = g or a

<220>
<221> misc_feature
<222> (193)..(193)
<223> n = a, t, g or c

<400> 51

```
gcatgccntg tccctcatgc cctttcctct cctttaccct ctgcagagct gagtgaggtg      6
caacagcagc tgctggccat gtccagggag aagggatcc tttcccaata ggtggagctg      12
gagagggncc tgggggatga caacgaggag aaaattgagt ttgagctcat ccagctccac     18
ccccacactt gtngatgggc agtctgaggc ccagagagca cacaagactt tagggccaca     24
cagcaggctc ttagc                                                      255
```

<210> 52
<211> 255
<212> DNA
<213> Homo sapiens

<220>
<221> misc_feature
<222> (63)..(63)
<223> n = a, t, g or c

<220>
<221> misc_feature
<222> (128)..(128)
<223> n = g or a

<400> 52

```
gcagctgctg gccatgtcca gggagaaggg gatcctttcc caataggtgg agctggagag      6
ggncctgggg gatgacaacg aggagaaaat tgagtttgag ctcatccagc tccaccccca     12
cacttgtnga tgggcagtct gaggcccaga gagcacacaa gactttaggg ccacacagca     18
ggctcttagc agaactctct acagaactta ggtctccgga ttcttaatcc ttgcccatct     24
ctgcttcact ctagc                                                      255
```

```
<210> 53
<211> 255
<212> DNA
<213> Homo sapiens


<220>
<221> misc_feature
<222> (23)..(23)
<223> n = a, t, g or c


<220>
<221> misc_feature
<222> (128)..(128)
<223> n = c or t


<400> 53
gaccccatct ctatttaata tangaaataa taaaaaataa aaagtaaaag aatggcagtg      6
tgatgccacc ttgaagcaaa actgccatgg tgactggtat tggattcctg attctggcag     12
caaagtcntt aaacaatgcc tgtagcatag ataacccctg tattagtcca ttctcacact     18
gctgtgaaga aatacctgag actgggtaat ttataaggga aagacattta attgactcac     24
agttccacat tgctg                                                     255


<210> 54
<211> 255
<212> DNA
<213> Homo sapiens


<220>
<221> misc_feature
<222> (80)..(80)
<223> n = a, t, g or c


<220>
<221> misc_feature
<222> (128)..(128)
<223> n = a or g


<220>
<221> misc_feature
<222> (236)..(236)
<223> n = a, t, g or c


<400> 54
gtgagggctg acaccttgaa gctggatgct gagtttgaca cacttagagc tgaacactga      6
tgtctgttca gccagagtcn ggggaggtct tggactccaa agctgatttg ggaatttggg     12
aagaaacntt aaagcttttt agagaataag gaaagagtat gggagcttta ctctgttcag     18
aaaggagaat ttatggaagt atagctcaca atctatgatc tgcaccccca aacatncaac     24
ctcccatatg agaga                                                     255


<210> 55
<211> 255
<212> DNA
<213> Homo sapiens
```

```
<220>
<221> misc_feature
<222> (20)..(20)
<223> n = a, t, g or c

<220>
<221> misc_feature
<222> (128)..(128)
<223> n = c or t

<220>
<221> misc_feature
<222> (175)..(175)
<223> n = a, t, g or c

<400> 55
tgggaatttg ggaagaaacn ttaaagcttt ttagagaata aggaaagagt atgggagctt      6
tactctgttc agaaaggaga atttatggaa gtatagctca caatctatga tctgcacccc     12
caaacatnca acctcccata tgagagataa aacacagcat ccctaggaag cattnttcct     18
gcgcctccac caggcagtca tgccccagcc cagcctgtct tcatccctaa ccattaccat     24
tccacctcct tttgt                                                      255


<210> 56
<211> 255
<212> DNA
<213> Homo sapiens


<220>
<221> misc_feature
<222> (81)..(81)
<223> n = a, t, g or c

<220>
<221> misc_feature
<222> (128)..(128)
<223> n = c or a

<220>
<221> misc_feature
<222> (210)..(210)
<223> n = a, t, g or c

<400> 56
agtatgggag ctttactctg ttcagaaagg agaatttatg gaagtatagc tcacaatcta      6
tgatctgcac ccccaaacat ncaacctccc atatgagaga taaaacacag catccctagg     12
aagcattntt cctgcgcctc caccaggcag tcatgcccca gcccagcctg tcttcatccc     18
taaccattac cattccacct cctttttgttn agggcaggtc tgggaatatg ttcatgtgag     24
ggatcacttc ttgca                                                      255


<210> 57
<211> 255
<212> DNA
<213> Homo sapiens


<220>
<221> misc_feature
<222> (46)..(46)
```

```
<223>  n = a, t, g or c


<220>
<221>  misc_feature
<222>  (128)..(128)
<223>  n = c or t


<220>
<221>  misc_feature
<222>  (249)..(249)
<223>  n = a, t, g or c


<400>  57
aacctcccat atgagagata aaacacagca tccctaggaa gcattnttcc tgcgcctcca        6
ccaggcagtc atgccccagc ccagcctgtc ttcatcccta accattacca ttccacctcc       12
ttttgttnag ggcaggtctg ggaatatgtt catgtgaggg atcacttctt gcaaatgctc       18
cagttatcct gcacccttca agtaggctcc aaatcctaca acattctcct tgccctccca       24
actcgcagng tgcca                                                       255


<210>  58
<211>  255
<212>  DNA
<213>  Homo sapiens


<220>
<221>  misc_feature
<222>  (7)..(7)
<223>  n = a, t, g or c

<220>
<221>  misc_feature
<222>  (128)..(128)
<223>  n = g or a


<400>  58
tttgttnagg gcaggtctgg gaatatgttc atgtgaggga tcacttcttg caaatgctcc        6
agttatcctg cacccttcaa gtaggctcca aatcctacaa cattctcctt gccctcccaa       12
ctcgcagngt gccagcttct tcccattctg gccagagttt gtgacttcca tgaacgccca       18
taatctactt ttctctgttt tctctgaccc catgctgagg gggaaatcaa caataataaa       24
aaggaggcca ggcac                                                       255


<210>  59
<211>  255
<212>  DNA
<213>  Homo sapiens


<220>
<221>  misc_feature
<222>  (128)..(128)
<223>  n = g or a

<220>
<221>  misc_feature
<222>  (252)..(252)
<223>  n = a, t, g or c

<400>  59
```

```
gcctatatcc cgggctcctt ggcagccttg gcagccacct tgacactctc ctgtctcccc      6
acctccacag agacaatgac catgtttgaa aatgtcaccc gggccctggc cagacagcta     12
aaccctcnag gggacctgac accacttgac agcctcatcg acttcaagcg cttccatccc     18
ttctgcctgg tgctgaggaa gaggaagagc acgctcttct ggggggcccg gtacgtccgc     24
accgactaca cnctg                                                      255
```

```
<210> 60
<211> 255
<212> DNA
<213> Homo sapiens


<220>
<221> misc_feature
<222> (128)..(128)
<223> n = g or t

<220>
<221> misc_feature
<222> (134)..(134)
<223> n = a, t, g or c

<220>
<221> misc_feature
<222> (156)..(156)
<223> n = a, t, g or c

<220>
<221> misc_feature
<222> (170)..(170)
<223> n = a, t, g or c

<400> 60
```

```
ggacacccga gtggagggag atgtggatgt accaaagacg gtgaaggtga agggaacggc      6
agggctctcg cagaacagca ctctggaggt ccagacactc agtgtggctc ccaaggccct     12
ggagaccntg cagnagaggt gagagtgggc gggacngagg gtctcccggn atgtgggtgg     18
ggcagtgcat ggaaatggtg cttggggcct tgagctgaaa ggtaggaccc ttgcactgac     24
ctttgccaat gtggt                                                      255
```

```
<210> 61
<211> 255
<212> DNA
<213> Homo sapiens


<220>
<221> misc_feature
<222> (15)..(15)
<223> n = a, t, g or c

<220>
<221> misc_feature
<222> (50)..(50)
<223> n = a, t, g or c

<220>
<221> misc_feature
<222> (107)..(107)
<223> n = a, t, g or c
```

```
<220>
<221> misc_feature
<222> (128)..(128)
<223> n = c or t


<220>
<221> misc_feature
<222> (237)..(237)
<223> n = a, t, g or c


<400> 61
ccttcctcac ctagnagggc atccaggaag tccagaatgg cttttgcacn cgcttctacc      6
aagaccccag cagcattaaa aaggctgctt aggagaggct tgtctgngtc ctccatgtgt     12
agctcccngg aaatcaggac ctcagatacc tagggccagg aagtgtggga gatgagcagc     18
agtctcacca tagcagatta tcctcactgt gccaactgcc atccctctg gctcctntct      24
ctctctgccc caagg                                                     255



<210> 62
<211> 255
<212> DNA
<213> Homo sapiens



<220>
<221> misc_feature
<222> (36)..(36)
<223> n = a, t, g or c


<220>
<221> misc_feature
<222> (71)..(71)
<223> n = a, t, g or c


<220>
<221> misc_feature
<222> (128)..(128)
<223> n = g or a


<220>
<221> misc_feature
<222> (149)..(149)
<223> n = a, t, g or c


<400> 62
cacccccagcc tacagcgaga cccttcctca cctagnaggg catccaggaa gtccagaatg     6
gcttttgcac ncgcttctac caagacccca gcagcattaa aaaggctgct taggagaggc     12
ttgtctgngt cctccatgtg tagctcccng gaaatcagga cctcagatac ctagggccag     18
gaagtgtggg agatgagcag cagtctcacc atagcagatt atcctcactg tgccaactgc     24
catcccctct ggctc                                                    255



<210> 63
<211> 255
<212> DNA
<213> Homo sapiens



<220>
<221> misc_feature
```

```
<222> (128)..(128)
<223> n = g or a

<220>
<221> misc_feature
<222> (170)..(170)
<223> n = a, t, g or c

<400> 63
gaggagggtg gggggtggta acagaagggg accccctgta tccctaaggc ctgtggtccc     6
tggtctccca ggtagacaga gggcttcagg acccagccag gcagctgggg atgtggggag    12
gaggctgntg gggaggaaac aggcagctgt cctcccggta gagctaagan cacactcagt    18
tccaccccag ttcagctcca ggaggtagag gggctgccca ctctcgagtc ccagatgctg    24
ggaggccttc cctag                                                    255


<210> 64
<211> 19
<212> RNA
<213> Artificial sequence sequence

<220>
<223> siRNA

<400> 64
cuaaguacga ccagaucca                                                 19


<210> 65
<211> 19
<212> RNA
<213> Artificial sequence

<220>
<223> siRNA

<400> 65
aaggcaugug cugcaacac                                                 19


<210> 66
<211> 19
<212> RNA
<213> Artificial sequence

<220>
<223> siRNA

<400> 66
agaagaagcc ucuggacac                                                 19


<210> 67
<211> 19
<212> RNA
<213> Artificial sequence

<220>
<223> siRNA

<400> 67
```

```
guagccaacu uggaguagc                                          19


<210> 68
<211> 19
<212> RNA
<213> Artificial sequence

<220>
<223> siRNA

<400> 68
ucaauaagua cugagagug                                          19


<210> 69
<211> 19
<212> RNA
<213> Artificial sequence

<220>
<223> siRNA

<400> 69
uaaguacuga gagugcagc                                          19


<210> 70
<211> 19
<212> RNA
<213> Artificial sequence

<220>
<223> siRNA

<400> 70
aguucuugac caucacacc                                          19
```